# EUROPÄISCHE PATENTANMELDUNG

(11) **EP 3 138 863 A1**
(43) Veröffentlichungstag der Anmeldung: **08.03.2017**
(21) Anmeldenummer: 15183276.3
(22) Anmeldetag: 01.09.2015
(51) Int. Cl.: C08G 59/18, C08G 59/50, C09D 163/00

(54) **EMISSIONSARME EPOXIDHARZ-ZUSAMMENSETZUNG**

(71) Anmelder: Sika Technology AG, 6340 Baar (CH)
(72) Erfinder: Kasemi, Edis, 8046 Zürich (CH); Kramer, Andreas, 8006 Zürich (CH); Stadelmann, Ursula, 8046 Zürich (CH); Burckhardt, Urs, 8049 Zürich (CH)
(74) Vertreter: Sika Patent Attorneys

(57) **Zusammenfassung**

Die vorliegende Erfindung betrifft eine Epoxidharz-Zusammensetzung, deren Härter-Komponente mindestens ein Amin der Formel (I) und gegebenenfalls mindestens ein Amin **A**, welches insbesondere ein Addukt aus einem Polyamin und einem Epoxid ist, enthält. Das Amin der Formel (I) wird insbesondere in Form eines Reaktionsprodukts aus der reduktiven Alkylierung von 1,2-Ethylendiamin und einem Aldehyd oder Keton eingesetzt.

Die Epoxidharz-Zusammensetzung ist insbesondere als emissionsarme raumtemperaturhärtende Epoxidharz-Beschichtung verwendbar. Sie zeichnet sich durch eine gute Verarbeitbarkeit, eine schnelle Aushärtung, eine hohe Härte, eine schöne Oberfläche und eine geringe Vergilbungsneigung aus.

## Beschreibung

### Technisches Gebiet

Die Erfindung betrifft das Gebiet der Härter für Epoxidharze, Epoxidharz-Zusammensetzungen, sowie deren Verwendung, insbesondere als Beschichtung, Belag oder Anstrich.

### Stand der Technik

Für Beschichtungszwecke geeignete Epoxidharz-Zusammensetzungen sollen eine möglichst niedrige Viskosität aufweisen, damit sie bei Umgebungstemperatur gut verarbeitbar sind. Weiterhin sollen sie möglichst schnell und störungsfrei aushärten, auch bei feucht-kalten Bedingungen, und dabei eine ebenmässige Oberfläche ohne Trübungen, Flecken oder Krater ausbilden. Schliesslich soll eine ausgehärtete Beschichtung eine hohe Härte bei geringer Sprödigkeit besitzen, um mechanischer Beanspruchung möglichst gut zu widerstehen. Für optisch anspruchsvolle Anwendungen, beispielsweise Deckbeläge von Fussböden, soll eine Beschichtung ausserdem einen hohen Glanzgrad und eine möglichst geringe Neigung zum Vergilben unter Lichteinfluss aufweisen.

Aus dem Stand der Technik bekannte Epoxidharz-Beschichtungen enthalten als Bestandteil der Härter-Komponente typischerweise Addukte von Polyaminen mit Epoxiden, insbesondere mit Bisphenol-Flüssigharzen. Solche Addukte ermöglichen eine rasche Aushärtung, sind aber sehr hochviskos, weshalb die Härter-Komponente zur Einstellung einer handhabbaren Viskosität zusätzlich üblicherweise beträchtliche Anteile an nicht adduktierten Polyaminen und/oder Verdünnern enthalten. Die nicht adduktierten Polyamine sind typischerweise geruchsintensiv und führen zu verstärktem Auftreten von Blushing-Effekten. Als "Blushing" werden bei der Aushärtung auftretende Oberflächenmängel wie Trübungen, Flecken, Rauheit oder Klebrigkeit bezeichnet, welche durch Salzbildung von Aminen mit Kohlendioxid (CO₂) aus der Luft verursacht werden und besonders bei hoher Luftfeuchtigkeit und tiefen Temperaturen auftreten. Verdünner vermindern typischerweise Blushing-Effekte und verbessern Oberflächenqualität und Sprödigkeit der Beschichtung. Da sie bei der Aushärtung nicht in die Harzmatrix eingebaut werden, können sie durch Verdampfungsoder Diffusionsprozesse in die Umgebung freigesetzt werden. Heutzutage werden aber zunehmend emissionsarme Produkte gewünscht, die nach der Aushärtung einen geringen Gehalt an freisetzbaren Substanzen aufweisen. Für emissionsarme Epoxidharz-Zusammensetzungen können Verdünner, wie beispielsweise Benzylalkohol, deshalb nur in geringer Menge oder gar nicht verwendet werden.

Aus US 2014/0107313 und EP 2 752 403 sind Amine bekannt, welche Epoxidharz-Zusammensetzungen gut verdünnen und kaum zu Blushing-Effekten neigen. Hinsichtlich Aushärtungsgeschwindigkeit und/oder Vergilbung der damit erhaltenen Epoxidharz-Zusammensetzungen sind diese Amine aber noch verbesserungsfähig.

### Darstellung der Erfindung

Aufgabe der vorliegenden Erfindung ist es daher, eine raumtemperaturhärtende Epoxidharz-Zusammensetzung zur Verfügung zu stellen, welche geruchsarm und niedrigviskos ist und emissionsarme Beschichtungen mit guter Verarbeitbarkeit ermöglicht, welche schnell aushärten und Beschichtungen von hoher Härte, guter Oberflächenqualität und geringer Vergilbungsneigung ergeben. Diese Aufgabe wird mit einer Epoxidharz-Zusammensetzung wie in Anspruch 1 beschrieben gelöst. Deren Härter-Komponente ist geruchsarm und so niedrigviskos, dass sie ohne Lösemittel oder Verdünner eingesetzt werden kann. Sie ist mit der Harz-Komponente überraschend gut verträglich und verdünnt diese stark. Die Epoxidharz-Zusammensetzung weist eine hohe Aushärtungsgeschwindigkeit auf, bleibt aber überraschenderweise trotzdem weitgehend frei von Blushing-Effekten, auch unter ungünstigen Aushärtungsbedingungen. Damit sind emissionsarme Epoxidharz-Beschichtungen mit ausgezeichneter Verarbeitbarkeit zugänglich, die schnell aushärten, eine hohe Endhärte und eine überraschend glänzende, ebenmässige und nichtklebrige Oberfläche ohne Trübungen, Flecken oder Krater aufweisen und überraschenderweise unter Lichteinfluss kaum vergilben.

Besonders ausgeprägt sind die vorteilhaften Eigenschaften der in Anspruch 1 beschriebenen Epoxidharz-Zusammensetzung, wenn die Härter-Komponente zusätzlich ein Addukt aus Polyaminen und Epoxiden enthält.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist eine Epoxidharz-Zusammensetzung umfassend
- eine Harz-Komponente enthaltend mindestens ein Epoxidharz, und
- eine Härter-Komponente enthaltend mindestens ein Amin der Formel (I), 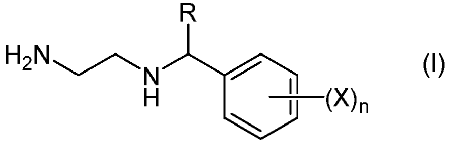 wobei
   n für 0 oder 1 oder 2 oder 3 steht,
   R für einen Wasserstoff-Rest oder für einen Kohlenwasserstoff-Rest mit 1 bis 6 C-Atomen steht, und
   X für gleiche oder verschiedene Reste ausgewählt aus der Gruppe bestehend aus Alkyl, Alkoxy und Dialkylamino mit jeweils 1 bis 18 C-Atomen steht,
   wobei die Härter-Komponente für den Fall, dass n für 0 steht, zusätzlich mindestens ein Amin **A** mit mindestens drei Aminwasserstoffen und einem Molekulargewicht von mindestens 200 g/mol, welches nicht der Formel (I) entspricht, enthält.

Als "Aminwasserstoff" werden die Wasserstoffatome von primären und sekundären Aminogruppen bezeichnet.
Als "Aminwasserstoff-Equivalentgewicht" wird die Masse eines Amins oder einer Amin-haltigen Zusammensetzung, die ein Molequivalent Aminwasserstoff enthält, bezeichnet.

Mit "Poly" beginnende Substanznamen wie Polyamin, Polyol oder Polyepoxid bezeichnen Substanzen, die formal zwei oder mehr der in ihrem Namen vorkommenden funktionellen Gruppen pro Molekül enthalten.
Als "primäre Aminogruppe" wird eine NH₂-Gruppe bezeichnet, die an einen organischen Rest gebunden ist und als "sekundäre Aminogruppe" wird eine NH-Gruppe bezeichnet, die an zwei organische Reste, welche auch gemeinsam Teil eines Rings sein können, gebunden ist.
Als "Verdünner" wird eine in einem Epoxidharz lösliche und dessen Viskosität senkende Substanz bezeichnet, welche bei der Aushärtung des Epoxidharzes nicht kovalent in die Harzmatrix eingebaut wird.
Mit dem Begriff "Viskosität" wird im vorliegenden Dokument die dynamische Viskosität oder Scherviskosität bezeichnet, welche durch das Verhältnis zwischen der Schubspannung und der Scherrate (Geschwindigkeitsgefälle) definiert ist und wie in den Ausführungsbeispielen beschrieben bestimmt wird. Unter "Molekulargewicht" versteht man im vorliegenden Dokument die molare Masse (in Gramm pro Mol) eines Moleküls. Als "mittleres Molekulargewicht" wird das Zahlenmittel Mₙ einer oligomeren oder polymeren Mischung von Molekülen bezeichnet, welches üblicherweise mittels Gelpermeationschromatographie (GPC) gegen Polystyrol als Standard bestimmt wird.
Als "Raumtemperatur" wird eine Temperatur von 23 °C bezeichnet.

Die Härter-Komponente enthält mindestens ein Amin der Formel (I). Bevorzugt steht R für einen Wasserstoff-Rest oder für Methyl oder für Phenyl. Diese Amine der Formel (I) sind besonders einfach zugänglich.
Besonders bevorzugt steht R für einen Wasserstoff-Rest oder für Methyl, insbesondere für einen Wasserstoff-Rest. Diese Amine der Formel (I) sind besonders einfach zugänglich und ermöglichen besonders niedrigviskose Härter-Komponenten und Epoxidharz-Zusammensetzungen.

Bevorzugt steht n für 0 oder 1 oder 2, insbesondere für 0 oder 1. Diese Amine ermöglichen besonders niedrigviskose Härter-Komponenten und Epoxidharz-Zusammensetzungen.

Ein Amin der Formel (I), bei welchem n für 0 steht, ist besonders kostengünstig und ermöglicht ganz besonders niedrigviskose Härter-Komponenten und Epoxidharz-Zusammensetzungen.
Ein Amin der Formel (I), bei welchem n für 1 steht, ist besonders geruchsarm und kann, je nach Gruppe X, eine besonders schnelle Aushärtung und/oder eine besonders gute Verträglichkeit in der Epoxidharz-Zusammensetzung ermöglichen.
Am meisten bevorzugt steht n für 0.

Bevorzugt steht X für gleiche oder verschiedene Reste ausgewählt aus der Gruppe bestehend aus Alkyl, Alkoxy und Dialkylamino mit jeweils 1 bis 12, insbesondere 1 bis 4, C-Atomen. Besonders bevorzugt steht X für Methyl oder Isopropyl oder tert.Butyl oder Methoxy oder Dimethylamino. Ganz besonders bevorzugt steht X für Methoxy oder Dimethylamino.
Bevorzugt steht der Rest X in meta- und/oder para-Stellung. Im Fall von n=1 steht der Rest X insbesondere in para-Stellung.

Besonders bevorzugt ist ein Amin der Formel (I), bei welchem R für einen Wasserstoff-Rest und n für 0 stehen. Dieses Amin der Formel (I) ist besonders gut zugänglich, besonders kostengünstig und besonders niedrigviskos. Es ermöglicht geruchs- und emissionsarme Epoxidharz-Zusammensetzungen mit besonders niedriger Viskosität und schneller Härteentwicklung bzw. Aushärtung, die auch bei feucht-kalten Bedingungen kaum Blushing-bedingte Oberflächenstörungen zeigen und praktisch nicht vergilben.

Besonders bevorzugt ist weiterhin ein Amin der Formel (I), bei welchem R für einen Wasserstoff-Rest, n für 1 und X für Methoxy oder Dimethylamino in para-Stellung stehen. Diese Amine der Formel (I) sind besonders geruchsarm, besonders verträglich und besonders reaktiv und ermöglichen besonders emissionsarme Epoxidharz-Zusammensetzungen mit besonders schneller Aushärtung und besonders schöner Oberfläche.

Besonders bevorzugte Amine der Formel (I) sind ausgewählt aus der Gruppe bestehend aus N-Benzyl-1,2-ethandiamin, N-(4-Methylbenzyl-1,2-ethandiamin, N-(4-Isopropylbenzyl)-1,2-ethandiamin, N-(4-tert.Butylbenzyl)-1,2-ethandiamin, N-(4-Methoxybenzyl)-1,2-ethandiamin, N-(4-(Dimethylamino)benzyl)-1,2-ethan-diamin, N-(1-Phenylethyl)-1,2-ethandiamin, N-Benzhydryl-1,2-ethandiamin, N-(1-(4'-Methyl)phenylethyl)-1,2-ethandiamin und N-(1-(4'-Methoxy)phenylethyl)-1,2-ethandiamin.

Davon bevorzugt ist N-Benzyl-1,2-ethandiamin, N-(4-Methoxybenzyl)-1,2-ethandiamin oder N-(4-(Dimethylamino)benzyl)-1,2-ethandiamin, insbesondere N-Benzyl-1,2-ethandiamin.

Das Amin der Formel (I) wird bevorzugt erhalten aus der einfachen Alkylierung von 1,2-Ethylendiamin mit einem geeigneten Alkylierungsmittel, beispielsweise einem organischen Halogenid oder einer Carbonylverbindung.
Bevorzugt wird das Amin der Formel (I) durch reduktive Alkylierung von 1,2-Ethylendiamin mit einem Aldehyd oder Keton der Formel (II) und Wasserstoff hergestellt.

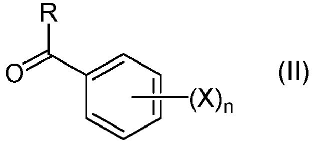

In der Formel (II) weisen R, X und n die bereits genannten Bedeutungen auf. Diese Herstellung verläuft besonders selektiv und führt zu Reaktionsprodukten von besonders hoher Reinheit, d.h. hohem Gehalt an Aminen der Formel (I).

Das Amin der Formel (I) wird somit bevorzugt in Form eines Reaktionsprodukts aus der reduktiven Alkylierung von 1,2-Ethylendiamin mit mindestens einem Aldehyd oder Keton der Formel (II) und Wasserstoff eingesetzt.
Ein solches Reaktionsprodukt ist auch ohne aufwendige Reinigungsschritte besonders rein, d.h. es enthält einen hohen Gehalt an Amin der Formel (I). Es ist dadurch besonders niedrigviskos und reaktiv und somit besonders geeignet als Bestandteil der beschriebenen Epoxidharz-Zusammensetzung.

Als Aldehyd der Formel (II) geeignet sind insbesondere Benzaldehyd, 2-Methylbenzaldehyd (o-Tolualdehyd), 3-Methylbenzaldehyd (m-Tolualdehyd), 4-Methylbenzaldehyd (p-Tolualdehyd), 2,5-Dimethylbenzaldehyd, 4-Ethylbenzaldehyd, 4-Isopropylbenzaldehyd (Cuminaldehyd), 4-tert.Butylbenzaldehyd, 2-Methoxybenzaldehyd (o-Anisaldehyd), 3-Methoxybenzaldehyd (m-Anisaldehyd), 4-Methoxybenzaldehyd (Anisaldehyd), 2,3-Dimethoxybenzaldehyd, 2,4-Dimethoxybenzaldehyd, 2,5-Dimethoxybenzaldehyd, 3,4-Dimethoxybenzaldehyd (Veratrumaldehyd), 3,5-Dimethoxybenzaldehyd, 2,4,6-Trimethylbenzaldehyd, 2,4,5-Trimethoxybenzaldehyd (Asaronaldehyd), 2,4,6-Trimethoxybenzaldehyd, 3,4,5-Trimethoxybenzaldehyd oder 4-Dimethylaminobenzaldehyd. Bevorzugt sind Benzaldehyd, 4-Isopropylbenzaldehyd (Cuminaldehyd), 4-tert.Butylbenzaldehyd, 4-Methoxybenzaldehyd (Anisaldehyd) oder 4-Dimethylaminobenzaldehyd.
Als Keton der Formel (II) geeignet sind insbesondere Acetophenon, Benzophenon, 2'-Methylacetophenon, 3'-Methylacetophenon, 4'-Methylacetophenon, 2'-Methoxyacetophenon, 3'-Methoxyacetophenon, 4'-Methoxyacetophenon, 2',4'-Dimethylacetophenon, 2',5'-Dimethylacetophenon, 3',4'-Dimethylacetophenon, 3',5'-Dimethylacetophenon, 2',4'-Dimethoxyacetophenon, 2',5'-Dimethoxyacetophenon, 3',4'-Dimethoxyacetophenon, 3',5'-Dimethoxyacetophenon, 2',4',6'-Trimethylacetophenon oder 2',4',6'-Trimethoxyacetophenon. Bevorzugt sind Acetophenon, Benzophenon, 4'-Methylacetophenon oder 4'-Methoxyacetophenon. Besonders bevorzugt ist Acetophenon.

Als Aldehyd oder Keton der Formel (II) besonders bevorzugt ist Benzaldehyd, 4-Methoxybenzaldehyd (Anisaldehyd) oder 4-Dimethylaminobenzaldehyd. Am meisten bevorzugt ist Benzaldehyd.

In einer Ausführungsform wird eine Mischung aus zwei oder mehr verschiedenen Aldehyden oder Ketonen der Formel (II) für die Umsetzung verwendet, insbesondere eine Mischung aus Benzaldehyd und 4-Methoxybenzaldehyd oder 4-Dimethylaminobenzaldehyd.

Die reduktive Alkylierung kann direkt mit molekularem Wasserstoff oder indirekt durch Wasserstoff-Transfer von anderen Reagentien, wie beispielsweise Ameisensäure, erfolgen. Bevorzugt wird molekularer Wasserstoff verwendet. Dabei werden die Bedingungen vorteilhaft so gewählt, dass vor allem jeweils eine primäre Aminogruppe von 1,2-Ethylendiamin mit guter Selektivität einfach alkyliert und der Benzolring nicht hydriert wird.
Bevorzugt wird die Umsetzung bei einer Temperatur von 40 bis 120 °C und in Anwesenheit eines geeigneten Katalysators durchgeführt. Als Katalysator bevorzugt sind Palladium auf Kohle (Pd/C), Platin auf Kohle (Pt/C), Adams-Katalysator oder Raney-Nickel, insbesondere Palladium auf Kohle oder Raney-Nickel.
Bei Verwendung von molekularem Wasserstoff wird bevorzugt in einer Druckapparatur bei einem Wasserstoff-Druck von 5 bis 150 bar, insbesondere 10 bis 100 bar, gearbeitet.

Das Reaktionsprodukt aus der beschriebenen reduktiven Alkylierung kann neben mindestens einem Amin der Formel (I) weitere Amine als Nebenprodukte enthalten. Als Nebenprodukt tritt hauptsächlich mehrfach alkyliertes 1,2-Ethylendiamin, insbesondere N,N'-dialkyliertes 1,2-Ethylendiamin oder N,N-dialkyliertes 1,2-Ethylendiamin, wie in den untenstehenden Formeln gezeichnet, auf. Die Anwesenheit solcher Nebenprodukte erhöht die Viskosität und senkt die Reaktivität des Reaktionsproduktes. Die Umsetzung wird deshalb bevorzugt so geführt, dass die Bildung von Nebenprodukten möglichst unterdrückt wird.

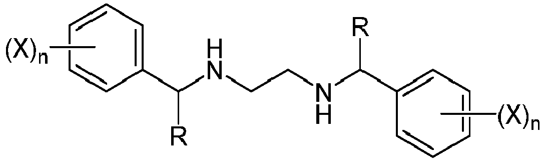

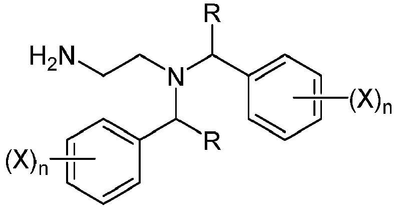

Die reduktive Alkylierung wird bevorzugt mit stöchiometrischem Überschuss von 1,2-Ethylendiamin gegenüber den Carbonylgruppen des Aldehyds oder Ketons der Formel (II) durchgeführt. Das Verhältnis zwischen der Anzahl 1,2-Ethylendiamin-Moleküle und der Anzahl Carbonylgruppen beträgt bevorzugt mindestens 2/1, insbesondere mindestens 3/1, besonders bevorzugt mindestens 4/1. Überschüssiges 1,2-Ethylendiamin wird vor oder bevorzugt nach der Reduktion entfernt, insbesondere durch Destillation, beispielsweise mittels Dünnschicht-, Kurzweg- oder Fallstromverfahren.
Auf diese Weise wird die Bildung von höher alkyliertem 1,2-Ethylendiamin zurückgedrängt, wodurch ein besonders niedrigviskoses und reaktives Reaktionsprodukt erhalten wird.

Das Amin der Formel (I) wird somit bevorzugt in Form eines Reaktionsprodukts aus der reduktiven Alkylierung von 1,2-Ethylendiamin mit mindestens einem Aldehyd oder Keton der Formel (II) und Wasserstoff eingesetzt, wobei 1,2-Ethylendiamin gegenüber den Carbonylgruppen des Aldehyds oder Ketons der Formel (II) im stöchiometrischen Überschuss eingesetzt und der Überschuss nach der Reduktion destillativ entfernt wird.

Bevorzugt ist das Reaktionsprodukt weitgehend frei von 1,2-Ethylendiamin. Es enthält insbesondere weniger als 1 Gewichts-%, bevorzugt weniger als 0.5 Gewichts-%, besonders bevorzugt weniger als 0.1 Gewichts-%, 1,2-Ethylendiamin.

Besonders bevorzugt wird das Reaktionsprodukt mittels Destillation gereinigt. Dabei wird das Reaktionsprodukt destilliert und das erhaltene Destillat verwendet. Ein solches mittels Destillation gereinigtes Reaktionsprodukt ermöglicht Epoxidharz-Zusammensetzung mit einer besonders schnellen Aushärtung.

Ganz besonders bevorzugt ist mittels Destillation gereinigtes N-Benzyl-1,2-ethandiamin aus der reduktiven Alkylierung von 1,2-Ethylendiamin mit Benzaldehyd, wobei 1,2-Ethylendiamin insbesondere im stöchiometrischen Überschuss gegenüber Benzaldehyd eingesetzt wurde. Ein solches mittels Destillation gereinigtes Reaktionsprodukt ermöglicht geruchs- und emissionsarme Epoxidharz-Zusammensetzungen mit sehr niedriger Viskosität, schneller Härteentwicklung bzw. Aushärtung und überraschend hoher Härte, welche überraschenderweise praktisch nicht vergilben.

Weiterhin ganz besonders bevorzugt ist mittels Destillation gereinigtes N-(4-Methoxybenzyl)-1,2-ethandiamin bzw. N-(4-(Dimethylamino)benzyl)-1,2-ethandiamin aus der reduktiven Alkylierung von 1,2-Ethylendiamin mit 4-Methoxybenzaldehyd (Anisaldehyd) bzw. 4-Dimethylaminobenzaldehyd, wobei 1,2-Ethylendiamin insbesondere im stöchiometrischen Überschuss gegenüber dem Aldehyd eingesetzt wurde. Ein solches mittels Destillation gereinigtes Reaktionsprodukt ermöglicht geruchs- und emissionsarme Epoxidharz-Zusammensetzungen mit niedriger Viskosität, sehr schneller Härteentwicklung bzw. Aushärtung und überraschend schöner Oberfläche.

Für den Fall, dass n für 0 steht, enthält die Härter-Komponente zusätzlich mindestens ein Amin **A** mit mindestens drei Aminwasserstoffen und einem Molekulargewicht von mindestens 200 g/mol, welches nicht der Formel (I) entspricht.
Das Amin **A** erhöht dabei insbesondere die Reaktivität der Härter-Komponente. Ohne Amin **A** ist die Aushärtungsgeschwindigkeit der Epoxidharz-Zusammensetzung unerwünscht niedrig, und unter feucht-kalten Bedingungen treten vermehrt Blushing-bedingte Oberflächenstörungen auf.

Für den Fall, dass n für 1 oder 2 oder 3 steht, enthält die Härter-Komponente bevorzugt ebenfalls zusätzlich mindestens ein Amin **A** wie beschrieben.

Ein weiterer Gegenstand der Erfindung ist somit eine Härter-Komponente enthaltend
- mindestens ein Amin der Formel (I), 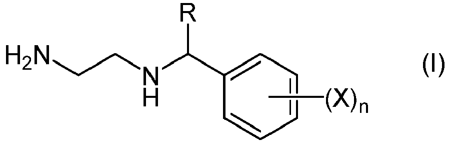 wobei
   n für 0 oder 1 oder 2 oder 3 steht,
   R für einen Wasserstoff-Rest oder für einen Kohlenwasserstoff-Rest mit 1 bis 6 C-Atomen steht, und
   X für gleiche oder verschiedene Reste ausgewählt aus der Gruppe bestehend aus Alkyl, Alkoxy und Dialkylamino mit jeweils 1 bis 18 C-Atomen steht,
- und mindestens ein Amin **A** mit mindestens drei Aminwasserstoffen und einem Molekulargewicht von mindestens 200 g/mol, welches nicht der Formel (I) entspricht.

Eine solche Härter-Komponente ist geruchsarm, weist eine niedrige Viskosität auf und bildet bei Luftkontakt kaum Trübungen oder Krusten. Gegenüber Epoxidharzen weist sie eine hohe Verdünnungswirkung bei guter Verträglichkeit und eine hohe Reaktivität auf. Sie ermöglicht somit emissionsarme Epoxidharz-Zusammensetzungen, welche gut verarbeitbar sind, besonders schnell und weitgehend ohne Blushing-Effekte aushärten und dabei Filme von hohem Glanz und hoher Härte bilden.

Als Amin **A** geeignet sind insbesondere die folgenden Polyamine:
- aliphatische, cycloaliphatische oder arylaliphatische primäre Diamine, insbesondere 1,12-Dodecandiamin, Bis(4-aminocyclohexyl)methan (H₁₂-MDA), Bis(4-amino-3-methylcyclohexyl)methan, Bis(4-amino-3-ethylcyclohexyl)methan, Bis(4-amino-3,5-dimethylcyclohexyl)methan oder Bis(4-amino-3-ethyl-5-methylcyclohexyl)methan;
- Ethergruppen-haltige aliphatische oder cycloaliphatische primäre Di- oder Triamine, insbesondere 4,9-Dioxadodecan-1,12-diamin, 5,8-Dioxadodecan-3,10-diamin, 4,7,10-Trioxatridecan-1,13-diamin oder höhere Oligomere dieser Diamine, 3,9-Bis(3-aminopropyl)-2,4,8,10-tetraoxaspiro[5.5]undecan, Bis(3-aminopropyl)polytetrahydrofurane oder andere Polytetrahydrofurandiamine, cycloaliphatische Ethergruppen-haltige Diamine aus der Propoxylierung und nachfolgenden Aminierung von 1,4-Dimethylolcyclohexan, erhältlich insbesondere als Jeffamine^{®} RFD-270 (von Huntsman), oder Polyoxyalkylendi- oder -triamine, welche typischerweise Produkte aus der Aminierung von Polyoxyalkylendi- oder -triolen darstellen und beispielsweise erhältlich sind unter dem Namen Jeffamine^{®} (von Huntsman), unter dem Namen Polyetheramine (von BASF) oder unter dem Namen PC Amine^{®} (von Nitroil). Insbesondere geeignete Polyoxyalkylendi- oder -triamine sind Jeffamine^{®} D-230, Jeffamine^{®} D-400, Jeffamine^{®} EDR-104, Jeffamine^{®} EDR-148, Jeffamine^{®} EDR-176 oder Jeffamine^{®} T-403, oder entsprechende Amine von BASF oder Nitroil;
- sekundäre Aminogruppen aufweisende Polyamine mit zwei primären aliphatischen Aminogruppen, wie insbesondere Bis(hexamethylen)triamin (BHMT), Pentaethylenhexamin (PEHA) oder höhere Homologe linearer Polyethylenamine wie Polyethylenpolyamin mit 5 bis 7 Ethylenamin-Einheiten (sogenanntes "higher ethylenepolyamine", HEPA), N,N'-Bis(3-aminopropyl)-1,4-diaminobutan, N5-(3-Amino-1-ethylpropyl)-2-methyl-1,5-pentandiamin oder N,N'-Bis(3-amino-1-ethylpropyl)-2-methyl-1,5-pentandiamin,
- Addukte von Polyaminen mit Epoxiden oder Epoxidharzen, insbesondere Addukte mit Diepoxiden im Molverhältnis von ungefähr 2/1, oder Addukte mit Monoepoxiden im Molverhältnis von ungefähr 1/1, oder Umsetzungsprodukte aus Polyaminen und Epichlorhydrin, insbesondere jenes von 1,3-Bis(amino-methyl)benzol, kommerziell erhältlich als Gaskamine^{®} 328 (von Mitsubishi Gas Chemical);
- Polyamidoamine, insbesondere Umsetzungsprodukte aus einer ein- oder mehrwertigen Carbonsäure, beziehungsweise deren Ester oder Anhydride, insbesondere einer Dimerfettsäure, mit einem im stöchiometrischen Überschuss eingesetzten aliphatischen, cycloaliphatischen oder aromatischen Polyamin, insbesondere einem Polyalkylenamin wie beispielsweise DETA oder TETA, insbesondere die kommerziell erhältlichen Polyamidoamine Versamid^{®} 100, 125, 140 oder 150 (von Cognis), Aradur^{®} 223, 250 oder 848 (von Huntsman), Euretek^{®} 3607 oder 530 (von Huntsman) oder Beckopox^{®} EH 651, EH 654, EH 655, EH 661 oder EH 663 (von Cytec); oder
- Phenalkamine, auch Mannich-Basen genannt, insbesondere Umsetzungsprodukte aus einer Mannich-Reaktion von Phenolen, insbesondere Cardanol, mit Aldehyden, insbesondere Formaldehyd, insbesondere die kommerziell erhältlichen Phenalkamine Cardolite^{®} NC-541, NC-557, NC-558, NC-566, Lite 2001, Lite 2002, NX-4943, NX-5607 oder NX-5608 (von Cardolite), Aradur^{®} 3440, 3441, 3442 oder 3460 (von Huntsman) oder Beckopox^{®} EH 614, EH 621, EH 624, EH 628 oder EH 629 (von Cytec).

Davon bevorzugt sind Addukte von Polyaminen mit Epoxiden, Polyamidoamine, Phenalkamine oder Ethergruppen-haltige aliphatische primäre Di- oder Triamine, insbesondere Polyoxyalkylendi- oder -triamine mit einem mittleren Molekulargewicht im Bereich von 200 bis 500 g/mol, insbesondere Jeffamine^{®} D-230 oder Jeffamine^{®} T-403 (beide von Huntsman), oder cycloaliphatische Ethergruppen-haltige Diamine aus der Propoxylierung und nachfolgenden Aminierung von 1,4-Dimethylolcyclohexan, insbesondere Jeffamine^{®} RFD-270 (von Huntsman).

Es kann vorteilhaft sein, wenn die Härter-Komponente eine Kombination aus mehreren Aminen **A** enthält.

Als Amin **A** besonders bevorzugt ist ein Addukt aus mindestens einem Polyamin mit 2 bis 12 C-Atomen und mindestens einem Epoxid.
Solche Addukte sind praktisch geruchsfrei und ermöglichen kostengünstige Epoxidharz-Zusammensetzungen mit schneller Aushärtung, hoher Härte und schöner Oberfläche. Sie sind aber ohne eine gute Verdünnung typischerweise zu hochviskos für viele Beschichtungsanwendungen.

Als Polyamin für ein solches Addukt geeignet sind insbesondere 1,2-Ethylendiamin, 1,2-Propylendiamin, 1,3-Propylendiamin, 1,2-Butandiamin, 1,3-Butandiamin, 1,4-Butandiamin, 2,3-Butandiamin, 2-Methyl-1,3-propandiamin, 2,2-Dimethyl-1,3-propandiamin, 1,3-Pentandiamin (DAMP), 1,5-Pentandiamin, 1,5-Diamino-2-methylpentan (MPMD), 1,6-Hexandiamin, 2,5-Dimethyl-1,6-hexandiamin, 2,2(4),4-Trimethylhexamethylendiamin (TMD), 1,7-Heptandiamin, 1,8-Octandiamin, 1,9-Nonandiamin, 1,10-Decandiamin, 1-Amino-3-aminomethyl-3,5,5-trimethylcyclohexan (Isophorondiamin oder IPDA), 1,2-Diaminocyclohexan, 1,3-Diaminocyclohexan, 1,4-Diaminocyclohexan, 2-oder4-Methyl-1,3-diaminocyclohexan oder Mischungen davon, 1,3-Bis(aminomethyl)cyclohexan, 1,4-Bis(aminomethyl)cyclohexan, 2,5(2,6)-Bis(aminomethyl)bicyclo[2.2.1]heptan (NBDA), 1,4-Diamino-2,2,6-trimethylcyclohexan (TMCDA), 1,8-Menthandiamin, 1,3-Bis(aminomethyl)benzol (MXDA), 1,4-Bis(aminomethyl)benzol, Bis-(2-aminoethyl)ether, 3,6-Dioxaoctan-1,8-diamin, 4,7-Dioxadecan-1,10-diamin, 4,7-Dioxadecan-2,9-diamin, 3-(2-Aminoethyl)aminopropylamin, Bis(hexamethylen)triamin (BHMT), Diethylentriamin (DETA), Triethylentetramin (TETA), Tetraethylenpentamin (TEPA), Pentaethylenhexamin (PEHA), Dipropylentriamin (DPTA), N-(2-Aminoethyl)-1,3-propandiamin (N3-Amin), N,N'-Bis(3-aminopropyl)ethylendiamin (N4-Amin), N,N'-Bis(3-aminopropyl)-1,4-diaminobutan, N5-(3-Aminopropyl)-2-methyl-1,5-pentandiamin oder N3-(3-Aminopentyl)-1,3-pentandiamin.
Davon bevorzugt ist 1,2-Ethylendiamin, 1,2-Propylendiamin, 1,3-Propylendiamin, 1,2-Butandiamin, 1,3-Butandiamin, 1,4-Butandiamin, DAMP, MPMD, TMD, IPDA, 2- oder 4-Methyl-1,3-diaminocyclohexan oder Mischungen davon, 1,3-Bis(aminomethyl)cyclohexan, MXDA, DETA, TETA, DPTA, N3-Amin, oder N4-Amin.
Diese Amine sind gut zugänglich und kostengünstig. Sie können in einer Epoxidharz-Zusammensetzung in unadduktierter Form aber Probleme mit Geruch und Emissionen und bei der Aushärtung Probleme mit Blushing-Effekten verursachen.
Bevorzugt enthält das in der Härter-Komponente enthaltene Addukt nur einen geringen Gehalt an solchen Polyaminen in unadduktierter Form.

Davon besonders bevorzugt ist 1,2-Ethylendiamin, 1,2-Propylendiamin oder MPMD. Diese Amine sind einfach zugänglich und sind nach der Adduktierung auf einfache Weise aus dem Addukt mittels Destillation entfernbar, wenn sie für die Adduktierung im Überschuss eingesetzt wurden. Die damit erhaltenen Addukte ermöglichen Epoxidharz-Zusammensetzungen mit schneller Aushärtung, hoher Härte und schönen Oberflächen.

Als Epoxid für ein solches Addukt bevorzugt sind aromatische Diepoxide, insbesondere Bisphenol-A- oder Bisphenol-F- oder Bisphenol-A/F-Diglycidylether oder Resorcinol-Diglycidylether, insbesondere kommerziell erhältliche Flüssigharze.

Als Epoxid für ein solches Addukt bevorzugt sind weiterhin Monoepoxide, insbesondere aromatische Monoepoxide, insbesondere Kresylglycidylether, tert.Butylphenylglycidylether oder der Glycidylether von Cardanol. Besonders bevorzugt ist Kresylglycidylether. Als Kresylglycidylether geeignet sind alle isomeren Kresylglycidylether oder Gemische davon, insbesondere kommerziell erhältliche Typen wie Araldite^{®} DY-K (von Huntsman), Polypox™ R6 (von Dow), Heloxy™ KR (von Hexion) oder Erisys^{®} GE-10 (von CVC Spec. Chem.).

Das Addukt wird bevorzugt hergestellt durch langsames Zudosieren des Epoxids zu vorgelegtem Polyamin, wobei die Temperatur der Reaktanden bevorzugt im Bereich von 40 bis 120 °C, insbesondere 50 bis 110 °C, gehalten wird.

Solche Addukte zeigen ausgezeichnete Eigenschaften als Härter für Epoxidharze, insbesondere eine schnelle Aushärtungsgeschwindigkeit auch bei tiefen Temperaturen und eine wenig ausgeprägte Neigung zu Blushing-Effekten. Sie ergeben Filme von ausgezeichneter Qualität, sind aber aufgrund ihrer Viskosität für Beschichtungsanwendungen nur geeignet, wenn sie verdünnt werden. Durch die Kombination mit dem Amin der Formel (I) entsteht eine Härter-Komponente, welche emissionsarme Epoxidharz-Beschichtungen mit ausgezeichneter Verarbeitbarkeit, schneller Aushärtung, schöner Oberfläche und geringer Neigung zu Vergilbung ermöglicht.

Insbesondere ist das Amin **A** ein Addukt aus mindestens einem Polyamin mit mindestens einem aromatischen Monoepoxid, welche im Molverhältnis von ungefähr 1/1 umgesetzt sind. Während der Umsetzung kann das Polyamin im Überschuss vorhanden gewesen und nach der Umsetzung mittels Destillation entfernt worden sein.

Für ein solches Addukt ist das aromatische Monoepoxid bevorzugt ein Kresylglycidylether, insbesondere ortho-Kresylglycidylether.
Für ein solches Addukt ist das Polyamin bevorzugt 1,2-Ethylendiamin, 1,2-Propylendiamin oder MPMD, besonders bevorzugt 1,2-Propylendiamin oder MPMD.

Ganz besonders bevorzugt ist das Amin A ein Addukt aus 1,2-Propylendiamin mit o-Kresylglycidylether, welches hergestellt ist mit einem Überschuss 1,2-Propylendiamin und nachfolgender Entfernung des Überschusses mittels Destillation. Ein solches Addukt enthält einen hohen Gehalt an 1-((2-Aminopropyl)-amino)-3-(2-methylphenoxy)propan-2-ol.

Weiterhin ganz besonders bevorzugt ist das Amin **A** ein Addukt aus 1,5-Diamino-2-methylpentan mit o-Kresylglycidylether, welches hergestellt ist mit einem Überschuss 1,5-Diamino-2-methylpentan und nachfolgender Entfernung des Überschusses mittels Destillation. Ein solches Addukt enthält einen hohen Gehalt an 1-((5-Amino-2(4)-methylpentyl)amino)-3-(2-methylphenoxy)propan-2-ol.

Der Begriff "Überschuss" bezieht sich dabei nicht auf die Reaktivgruppen, sondern auf das Molverhältnis zwischen dem Polyaminmolekül und dem Kresylglycidylether.

Diese ganz besonders bevorzugten Addukte sind vergleichsweise niedrigviskos, weisen eine besonders gute Verträglichkeit und Reaktivität mit den üblichen Epoxidharz-Zusammensetzungen auf, neigen kaum zu Blushing-Effekten und ermöglichen ausgehärtete Filme von hohem Glanz und hoher Härte. Allein eingesetzt sind aber auch diese Addukte zu hochviskos als Härter für Epoxidharz-Beschichtungen.

Weiterhin ist das Amin **A** insbesondere ein Addukt aus mindestens einem Polyamin und mindestens einem aromatischen Diepoxid, welche im Molverhältnis von ungefähr 2/1 umgesetzt sind. Während der Umsetzung kann das Polyamin im Überschuss vorhanden gewesen und nach der Umsetzung mittels Destillation entfernt worden sein.
Der Begriff "Überschuss" bezieht sich dabei nicht auf die Reaktivgruppen, sondern auf das Molverhältnis zwischen dem Polyamin-Molekül und dem Diepoxid-Molekül.

Für ein solches Addukt ist das aromatische Diepoxid bevorzugt ein Bisphenol-A- oder Bisphenol-F- oder Bisphenol-A/F-Diglycidylether oder ein Resorcinol-Diglycidylether, insbesondere ein kommerziell erhältliches Flüssigharz.

Für ein solches Addukt ist das Polyamin bevorzugt 1,2-Ethylendiamin, 1,2-Propylendiamin oder MPMD, insbesondere 1,2-Propylendiamin.

Diese Addukte sind einfach zugänglich und weisen eine besonders gute Verträglichkeit und Reaktivität mit den üblichen Epoxidharz-Zusammensetzungen auf, neigen kaum zu Blushing-Effekten und ermöglichen ausgehärtete Filme von hohem Glanz und hoher Härte. Allein eingesetzt sind sie aber viel zu hochviskos als Härter für Epoxidharz-Beschichtungen.

Die Härter-Komponente kann weitere gegenüber Epoxiden reaktive Amine enthalten, insbesondere die Folgenden:
- aliphatische, cacloaliphatische oder arylaliphatische Polyamine mit einem Molekulargewicht von weniger als 200 g/mol, insbesondere die bereits als geeignet zur Herstellung von Addukten genannten Polyamine, weiterhin Triamine wie insbesondere 4-Aminomethyl-1,8-octandiamin, 1,3,5-Tris(aminomethyl)benzol, 1,3,5-Tris(aminomethyl)cyclohexan, Tris(2-aminoethyl)amin, Tris(2-aminopropyl)amin oder Tris(3-aminopropyl)amin;
- Polyamine mit ein oder zwei sekundären Aminogruppen, insbesondere Produkte aus der reduktiven Alkylierung von primären aliphatischen Polyaminen mit Aldehyden oder Ketonen, insbesondere N-Benzyl-1,2-propandiamin, N,N'-Dibenzyl-1,2-propandiamin, N,N'-Dibenzyl-1,2-ethandiamin, N-Benzyl-1,3-bis(aminomethyl)benzol, N,N'-Dibenzyl-1,3-bis(aminomethyl)benzol, N-2-Ethylhexyl-1,3-bis(aminomethyl)benzol, N,N'-Bis(2-ethylhexyl)-1,3-bis(aminomethyl)benzol, oder partiell styrolisierte Polyamine wie zum Beispiel styrolisiertes MXDA (erhältlich als Gaskamine^{®} 240 von Mitsubishi Gas Chemical);
- aromatische Polyamine, wie insbesondere m- und p-Phenylendiamin, 4,4'-, 2,4' und/oder 2,2'-Diaminodiphenylmethan, 3,3'-Dichloro-4,4'-diaminodiphenylmethan (MOCA), 2,4- und/oder 2,6-Toluylendiamin, Mischungen von 3,5-Dimethylthio-2,4- und -2,6-toluylendiamin (erhältlich als Ethacure^{®} 300 von Albemarle), Mischungen von 3,5-Diethyl-2,4- und -2,6-toluylendiamin (DETDA), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 4,4'-Diaminodiphenylsulfon (DDS), 4-Amino-N-(4-aminophenyl)benzolsulfonamid, 5,5'-Methylendianthranilsäure, Dimethyl-(5,5'-methylendianthranilat), 1,3-Propylen-bis(4-aminobenzoat), 1,4-Butylen-bis(4-aminobenzoat), Polytetramethylenoxid-bis(4-aminobenzoat) (erhältlich als Versalink^{®} von Air Products), 1,2-Bis(2-aminophenylthio)ethan, 2-Methylpropyl-(4-chloro-3,5-diaminobenzoat) oder tert.Butyl-(4-chloro-3,5-diaminobenzoat).

Die Härter-Komponente ist bevorzugt weitgehend frei von Aminen mit einem Molekulargewicht unterhalb von 150 g/mol, insbesondere unterhalb von 120 g/mol. Bevorzugt enthält sie weniger als 2 Gewichts-%, insbesondere weniger als 1 Gewichts-%, Amine mit einem Molekulargewicht unterhalb von 120 g/mol, insbesondere unterhalb von 150 g/mol.
Eine solche Härter-Komponente ist toxikologisch und geruchlich besonders vorteilhaft und ermöglicht Beschichtungen mit besonders schönen Oberflächen.

Die Härter-Komponente kann weiterhin mindestens einen Beschleuniger enthalten. Als Beschleuniger geeignet sind Substanzen, welche die Reaktion zwischen Aminogruppen und Epoxidgruppen beschleunigen, insbesondere Säuren oder zu Säuren hydrolysierbare Verbindungen, insbesondere organische Carbonsäuren wie Essigsäure, Benzoesäure, Salicylsäure, 2-Nitrobenzoesäure, Milchsäure, organische Sulfonsäuren wie Methansulfonsäure, p-Toluolsulfonsäure oder 4-Dodecylbenzolsulfonsäure, Sulfonsäureester, andere organische oder anorganische Säuren wie insbesondere Phosphorsäure, oder Mischungen der vorgenannten Säuren und Säureester; tertiäre Amine wie insbesondere 1,4-Diazabicyclo[2.2.2]octan, Benzyldimethylamin, a-Methylbenzyldimethylamin, Triethanolamin, Dimethyl-aminopropylamin, Imidazole wie insbesondere N-Methylimidazol, N-Vinylimidazol oder 1,2-Dimethylimidazol, Salze solcher tertiärer Amine, quaternäre Ammoniumsalze, wie insbesondere Benzyltrimethylammoniumchlorid, Amidine wie insbesondere 1,8-Diazabicyclo[5.4.0]-undec-7-en, Guanidine wie insbesondere 1,1,3,3-Tetramethylguanidin, Phenole, insbesondere Bisphenole, Phenol-Harze oder Mannich-Basen wie insbesondere 2-(Dimethylaminomethyl)phenol, 2,4,6-Tris(dimethylaminomethyl)-phenol oder Polymere aus Phenol, Formaldehyd und N,N-Dimethyl-1,3-propandiamin, Phosphite wie insbesondere Di- oder Triphenylphosphite, oder Mercaptogruppen aufweisende Verbindungen. Als Beschleuniger bevorzugt sind Säuren, tertiäre Amine oder Mannich-Basen.
Am meisten bevorzugt ist Salicylsäure oder 2,4,6-Tris(dimethylaminomethyl)-phenol oder eine Kombination davon.

Die Härter-Komponente kann weiterhin mindestens einen Verdünner enthalten, insbesondere Xylol, 2-Methoxyethanol, Dimethoxyethanol, 2-Ethoxyethanol, 2-Propoxyethanol, 2-Isopropoxyethanol, 2-Butoxyethanol, 2-Phenoxyethanol, 2-Benzyloxyethanol, Benzylalkohol, Ethylenglykol, Ethylenglykoldimethylether, Ethylenglykoldiethylether, Ethylenglykoldibutylether, Ethylenglykoldiphenylether, Diethylenglykol, Diethylenglykol-monomethylether, Diethylenglykol-monoethylether, Diethylenglykol-mono-n-butylether, Diethylenglykoldimethylether, Diethylenglykoldiethylether, Diethylenglykoldi-n-butylylether, Propylenglykolbutylether, Propylenglykolphenylether, Dipropylenglykol, Dipropylenglykolmonomethylether, Dipropylenglykoldimethylether, Dipropylenglykoldi-n-butylether, N-Methylpyrrolidon, Diphenylmethan, Diisopropylnaphthalin, Erdölfraktionen wie zum Beispiel Solvesso^{®}-Typen (von Exxon), Alkylphenole wie tert.Butylphenol, Nonylphenol, Dodecylphenol und 8,11,14-Pentadecatrienylphenol (Cardanol, aus Cashewschalen-Öl, erhältlich beispielsweise als Cardolite NC-700 von Cardolite Corp., USA), styrolisiertes Phenol, Bisphenole, aromatische Kohlenwasserstoffharze, insbesondere Phenolgruppen-haltige Typen, alkoxyliertes Phenol, insbesondere ethoxyliertes oder propoxyliertes Phenol, insbesondere 2-Phenoxyethanol, Adipate, Sebacate, Phthalate, Benzoate, organische Phosphor- oder Sulfonsäureester oder Sulfonamide. Bevorzugt sind Benzylalkohol, Dodecylphenol, tert.Butylphenol, styrolisiertes Phenol, ethoxyliertes Phenol oder phenolgruppenhaltige aromatische Kohlenwasserstoffharze, insbesondere die Novares^{®}-Typen LS 500, LX 200, LA 300 oder LA 700 (von Rütgers).
Bevorzugt enthält die Härter-Komponente keinen oder nur einen geringen Gehalt an Verdünnern. Bevorzugt enthält sie maximal 5 Gewichts-% Verdünner.

Die Härter-Komponente kann weitere gegenüber Epoxidgruppen reaktive Substanzen enthalten, beispielsweise Monoamine wie Hexylamin oder Benzylamin, oder Mercaptogruppen aufweisende Verbindungen, insbesondere die Folgenden:
- flüssige Mercaptan-terminierte Polysulfid-Polymere, bekannt unter dem Markennamen Thiokol^{®} (von Morton Thiokol; beispielsweise erhältlich von SPI Supplies, oder von Toray Fine Chemicals), insbesondere die Typen LP-3, LP-33, LP-980, LP-23, LP-55, LP-56, LP-12, LP-31, LP-32 oder LP-2; sowie weiterhin bekannt unter dem Markennamen Thioplast^{®} (von Akzo Nobel), insbesondere die Typen G 10, G 112, G 131, G 1, G 12, G 21, G 22, G 44 oder G 4;
- Mercaptan-terminierte Polyoxyalkylen-Ether, erhältlich beispielsweise durch Umsetzung von Polyoxyalkylendi- oder -triolen entweder mit Epichlorhydrin oder mit einem Alkylenoxid, gefolgt von Natriumhydrogensulfid;
- Mercaptan-terminierte Verbindungen in Form von Polyoxyalkylen-Derivaten, bekannt unter dem Markennamen Capcure^{®} (von Cognis), insbesondere die Typen WR-8, LOF oder 3-800;
- Polyester von Thiocarbonsäuren, beispielsweise Pentaerythritoltetramercaptoacetat, Trimethylolpropantrimercaptoacetat, Glykoldimercaptoacetat, Pentaerythritoltetra-(3-mercaptopropionat), Trimethylolpropantri(3-mercaptopropionat) oder Glykoldi-(3-mercaptopropionat), oder Veresterungsprodukte von Polyoxyalkylendiolen oder -triolen, ethoxyliertem Trimethylolpropan oder Polyester-Diolen mit Thiocarbonsäuren wie Thioglykolsäure oder 2- oder 3-Mercaptopropionsäure; oder
- weitere Mercaptogruppen aufweisende Verbindungen, wie insbesondere 2,4,6-Trimercapto-1,3,5-triazin, 2,2'-(Ethylendioxy)-diethanthiol (Triethylenglykol-dimercaptan) oder Ethandithiol.

Die Härter-Komponente enthält bevorzugt 1 bis 90 Gewichts-%, bevorzugt 2 bis 80 Gewichts-%, besonders bevorzugt 5 bis 65 Gewichts-%, insbesondere 10 bis 50 Gewichts-%, Amin der Formel (I). Solche Härter-Komponenten zeichnen sich durch eine niedrige Viskosität aus und ermöglichen Epoxidharz-Beschichtungen mit hoher Aushärtungsgeschwindigkeit, kaum Neigung zu Blushing-Effekten und hoher Härte.

Eine besonders bevorzugte Härter-Komponente enthält
- mindestens ein Amin der Formel (I),
- mindestens ein Addukt, welches entweder ein Addukt aus mindestens einem Polyamin und mindestens einem aromatischen Monoepoxid, welche ungefähr im Molverhältnis von 1/1 umgesetzt sind, oder ein Addukt aus mindestens einem Polyamin und mindestens einem aromatischen Diepoxid, welche ungefähr im Molverhältnis von 2/1 umgesetzt sind, darstellt, und
- gegebenenfalls mindestens ein weiteres Amin, das nicht der Formel (I) entspricht, und/oder mindestens einen Beschleuniger.

Dabei liegen das Amin der Formel (I), das Addukt und das weitere Amin in einer solchen Menge vor, dass von den in der Härter-Komponente insgesamt enthaltenen Aminwasserstoffen
10 bis 80 % aus Aminen der Formel (I),
20 bis 80 % aus Addukten, und
0 bis 40 % aus weiteren Aminen stammen.

Eine solche Härter-Komponente ist geruchsarm, weist eine niedrige Viskosität auf und bildet bei Luftkontakt kaum Trübungen oder Krusten. Gegenüber Epoxidharzen weist sie eine hohe Verdünnungswirkung bei besonders guter Verträglichkeit und besonders hoher Reaktivität auf. Sie ermöglicht somit emissionsarme Epoxidharz-Zusammensetzungen, welche gut verarbeitbar sind, besonders schnell und weitgehend ohne Blushing-Effekte aushärten und dabei Filme von sehr hohem Glanz und hoher Härte bilden.

Das weitere Amin kann dabei ein Amin **A,** wie vorgängig beschrieben, darstelen.

Die Harz-Komponente der beschriebenen Epoxidharz-Zusammensetzung enthält mindestens ein Epoxidharz.

Als Epoxidharz sind übliche technische Epoxidharze geeignet. Diese werden auf bekannte Art und Weise erhalten, zum Beispiel aus der Oxidation der entsprechenden Olefine oder aus der Reaktion von Epichlorhydrin mit den entsprechenden Polyolen, Polyphenolen oder Aminen.
Als Epoxidharz besonders geeignet sind sogenannte Polyepoxid-Flüssigharze, im folgenden als "Flüssigharz" bezeichnet. Diese weisen eine Glasübergangstemperatur unterhalb von 25°C auf.
Ebenfalls möglich als Epoxidharz sind sogenannte Festharze, welche eine Glasübergangstemperatur oberhalb von 25°C aufweisen und sich zu bei 25°C schüttfähigen Pulvern zerkleinern lassen.

Geeignete Epoxidharze sind insbesondere aromatische Epoxidharze, insbesondere die Glycidylisierungsprodukte von:
- Bisphenol-A, Bisphenol-F oder Bisphenol-A/F, wobei A für Aceton und F für Formaldehyd steht, welche als Edukte zur Herstellung dieser Bisphenole dienten. Im Fall von Bisphenol-F können auch Stellungsisomere vorhanden sein, insbesondere abgeleitet von 2,4'- oder 2,2'-Hydroxyphenylmethan.
- Dihydroxybenzol-Derivaten wie Resorcin, Hydrochinon oder Brenzkatechin;
- weiteren Bisphenolen oder Polyphenolen wie Bis(4-hydroxy-3-methylphenyl)methan, 2,2-Bis(4-hydroxy-3-methylphenyl)propan (Bisphenol-C), Bis-(3,5-dimethyl-4-hydroxyphenyl)methan, 2,2-Bis(3,5-dimethyl-4-hydroxyphenyl)propan, 2,2-Bis(3,5-dibromo-4-hydroxyphenyl)propan, 2,2-Bis(4-hydroxy-3-tert.butylphenyl)propan, 2,2-Bis(4-hydroxyphenyl)butan (Bisphenol-B), 3,3-Bis(4-hydroxyphenyl)pentan, 3,4-Bis(4-hydroxyphenyl)hexan, 4,4-Bis(4-hydroxyphenyl)heptan, 2,4-Bis(4-hydroxyphenyl)-2-methylbutan, 2,4-Bis-(3,5-dimethyl-4-hydroxyphenyl)-2-methylbutan, 1,1-Bis(4-hydroxyphenyl)-cyclohexan (Bisphenol-Z), 1,1-Bis(4-hydroxyphenyl)-3,3,5-trimethylcyclohexan (Bisphenol-TMC), 1,1-Bis(4-hydroxyphenyl)-1-phenylethan, 1,4-Bis[2-(4-hydroxyphenyl)-2-propyl]benzol (Bisphenol-P), 1,3-Bis[2-(4-hydroxyphenyl)-2-propyl]benzol (Bisphenol-M), 4,4'-Dihydroxydiphenyl (DOD), 4,4'-Dihydroxybenzophenon, Bis(2-hydroxynaphth-1-yl)methan, Bis(4-hydroxynaphth-1-yl)methan, 1,5-Dihydroxynaphthalin, Tris(4-hydroxyphenyl)methan, 1,1,2,2-Tetrakis(4-hydroxyphenyl)ethan, Bis(4-hydroxyphenyl)ether oder Bis(4-hydroxyphenyl)sulfon;
- Kondensationsprodukten von Phenolen mit Formaldehyd, die unter sauren Bedingungen erhalten werden, wie Phenol-Novolaken oder Kresol-Novolaken, auch Bisphenol-F-Novolake genannt;
- aromatischen Aminen, wie Anilin, Toluidin, 4-Aminophenol, 4,4'-Methylendiphenyldiamin, 4,4'-Methylendiphenyldi-(N-methyl)amin, 4,4'-[1,4-Phenylen-bis(1-methylethyliden)]bisanilin (Bisanilin-P) oder 4,4'-[1,3-Phenylen-bis(1-methylethyliden)]bisanilin (Bisanilin-M).

Weitere geeignete Epoxidharze sind aliphatische oder cycloaliphatische Polyepoxide, insbesondere
- Glycidylether von gesättigten oder ungesättigten, verzweigten oder unverzweigten, zyklischen oder offenkettigen di-, tri- oder tetrafunktionellen C₂-bis C₃₀-Alkoholen, insbesondere Ethylenglykol, Propylenglykol, Butylenglykol, Hexandiol, Octandiol, Polypropylenglykolen, Dimethylolcyclohexan, Neopentylglykol, Dibromoneopentylglykol, Rizinusöl, Trimethylolpropan, Trimethylolethan, Pentaerythrol, Sorbit oder Glycerin, oder alkoxyliertes Glycerin oder alkoxyliertes Trimethylolpropan;
- ein hydriertes Bisphenol-A-, -F- oder -A/F-Flüssigharz, beziehungsweise die Glycidylisierungsprodukte von hydriertem Bisphenol-A, -F oder -A/F;
- ein N-Glycidylderivat von Amiden oder heterocyclischen Stickstoffbasen, wie Triglycidylcyanurat oder Triglycidylisocyanurat, oder Umsetzungsprodukte von Epichlorhydrin mit Hydantoin.
- Epoxidharze aus der Oxidation von Olefinen, wie insbesondere Vinylcylohexen, Dicyclopentadien, Cyclohexadien, Cyclododecadien, Cyclododecatrien, Isopren, 1,5-Hexadien, Butadien, Polybutadien oder Divinylbenzol.

Als Epoxidharz in der Harz-Komponente bevorzugt ist ein Flüssigharz auf der Basis eines Bisphenols, insbesondere ein Diglycidylether von Bisphenol-A, Bisphenol-F oder Bisphenol-A/F, wie sie kommerziell beispielsweise von Dow, Huntsman oder Momentive erhältlich sind. Diese Flüssigharze weisen eine für Epoxidharze niedrige Viskosität und im ausgehärteten Zustand gute Eigenschaften als Beschichtung auf. Sie können Anteile von Bisphenol A-Festharz oder Bisphenol-F-Novolaken enthalten.

Die Harz-Komponente kann einen Reaktivverdünner, insbesondere einen mindestens eine Epoxidgruppe aufweisenden Reaktivverdünner, enthalten. Als Reaktivverdünner geeignet sind insbesondere die Glycidylether von ein- oder mehrwertigen Phenolen oder aliphatischen oder cycloaliphatischen Alkoholen, wie insbesondere die bereits genannten Polyglycidylether von Di- oder Polyolen, oder weiterhin Phenylglycidylether, Kresylglycidylether, Benzylglycidylether, p-n-Butylphenylglycidylether, p-tert.Butylphenylglycidylether, Nonylphenylglycidylether, Allylglycidylether, Butylglycidylether, Hexylglycidylether, 2-Ethylhexylglycidylether, oder Glycidylether von natürlichen Alkoholen wie insbesondere C₈- bis C₁₀-Alkylglycidylether oder C₁₂- bis C₁₄-Alkylglycidylether. Die Zugabe eines Reaktivverdünners zum Epoxidharz bewirkt eine Reduktion der Viskosität, und/oder eine Reduktion der Glasübergangstemperatur und/oder der mechanischen Werte.

Gegebenenfalls enthält die Epoxidharz-Zusammensetzung weitere Bestandteile, insbesondere in Epoxidharz-Zusammensetzungen üblicherweise eingesetzte Hilfs- und Zusatzstoffe, beispielsweise die Folgenden:
- Lösemittel, Verdünner, Filmbildehilfsmittel oder Extender, wie insbesondere die bereits genannten Verdünner;
- Reaktivverdünner, insbesondere Epoxidgruppen aufweisende Reaktivverdünner, wie sie vorgängig erwähnt wurden, epoxidiertes Sojaöl oder Leinöl, Acetoacetatgruppen aufweisende Verbindungen, insbesondere acetoacetylierte Polyole, Butyrolakton, Carbonate, Aldehyde, sowie weiterhin Isocyanate oder Reaktivgruppen-aufweisende Silikone;

- Polymere, insbesondere Polyamide, Polysulfide, Polyvinylformal (PVF), Polyvinylbutyral (PVB), Polyurethane (PUR), Polymere mit Carboxylgruppen, Polyamide, Butadien-Acrylnitril-Copolymere, Styrol-Acrylnitril-Copolymere, Butadien-Styrol-Copolymere, Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere chlorsulfonierte Polyethylene oder Fluor-haltige Polymere, Sulfonamid-modifizierte Melamine oder gereinigte Montan-Wachse;
- anorganische oder organische Füllstoffe, insbesondere gemahlene oder gefällte Calciumcarbonate, welche gegebenenfalls mit Fettsäuren, insbesondere Stearaten, beschichtet sind, Baryt (Schwerspat), Talke, Quarzmehle, Quarzsand, Eisenglimmer, Dolomite, Wollastonite, Kaoline, Mica (Kalium-Aluminium-Silikat), Molekularsiebe, Aluminiumoxide, Aluminiumhydroxide, Magnesiumhydroxid, Kieselsäuren, Zemente, Gipse, Flugaschen, Russ, Graphit, Metall-Pulver wie Aluminium, Kupfer, Eisen, Zink, Silber oder Stahl, PVC-Pulver oder Hohlkugeln;
- Fasern, insbesondere Glasfasern, Kohlefasern, Metallfasern, Keramikfasern oder Kunststofffasern wie Polyamidfasern oder Polyethylenfasern;
- Pigmente, insbesondere Titandioxid und/oder Eisenoxide;
- die vorgenannten Beschleuniger;
- Rheologie-Modifizierer, insbesondere Verdicker oder Antiabsetzmittel;
- Haftverbesserer, insbesondere Organoalkoxysilane;
- Stabilisatoren gegen Oxidation, Wärme, Licht oder UV-Strahlung;
- flammhemmende Substanzen, insbesondere Aluminiumhydroxid (ATH), Magnesiumdihydroxid (MDH), Antimontrioxid, Antimonpentoxid, Borsäure (B(OH)₃), Zinkborat, Zinkphosphat, Melaminborat, Melamincyanurat, Ammoniumpolyphosphat, Melaminphosphat, Melaminpyrophosphat, polybromierte Diphenyloxide oder Diphenylether, Phosphate wie insbesondere Diphenylkresylphosphat, Resorcinol-bis(diphenylphosphat), Resorcinoldiphosphat-Oligomer, Tetraphenylresorcinoldiphosphit, Ethylendiamindiphosphat oder Bisphenol-A-bis(diphenylphosphat), Tris(chloroethyl)phosphat, Tris(chloropropyl)phosphat oder Tris(dichloroisopropyl)phosphat, Tris[3-bromo-2,2-bis-(bromomethyl)propyl]phosphat, Tetrabromo-Bisphenol-A, Bis(2,3-dibromopropylether) von Bisphenol A, bromierte Epoxidharze, Ethylen-bis(tetrabromophthalimid), Ethylen-bis(dibromonorbornandicarboximid), 1,2-Bis(tribromophenoxy)ethan, Tris(2,3-dibromopropyl)isocyanurat, Tribromophenol, Hexabromocyclododecan, Bis(hexachlorocyclopentadieno)cyclooctan oder Chlorparaffine;
- oberflächenaktive Substanzen, insbesondere Netzmittel, Verlaufsmittel, Entlüftungsmittel oder Entschäumer;
- Biozide, wie beispielsweise Algizide, Fungizide oder das Pilzwachstum hemmende Substanzen.

Bevorzugt enthält die Epoxidharz-Zusammensetzung weitere Hilfs- und Zusatzstoffe, insbesondere Netzmittel, Verlaufsmittel, Entschäumer, Stabilisatoren, Pigmente und/oder Beschleuniger, insbesondere Salicylsäure und/oder 2,4,6-Tris(dimethylaminomethyl)phenol.
Bevorzugt enthält die Epoxidharz-Zusammensetzung keinen oder nur einen geringen Gehalt an Verdünnern, bevorzugt maximal 5 Gewichts-%, insbesondere maximal 2 Gewichts-%.

In der Epoxidharz-Zusammensetzung liegt das Verhältnis der Anzahl von gegenüber Epoxidgruppen reaktiven Gruppen gegenüber der Anzahl Epoxidgruppen bevorzugt im Bereich von 0.5 bis 1.5, insbesondere 0.7 bis 1.2.
Die in der Epoxidharz-Zusammensetzung vorhandenen Aminwasserstoffe und gegebenenfalls vorhandene weitere gegenüber Epoxidgruppen reaktive Gruppen reagieren mit den Epoxidgruppen unter deren Ringöffnung (Additionsreaktion). Als Ergebnis dieser Reaktionen polymerisiert die Zusammensetzung und härtet schliesslich aus. Dem Fachmann ist bekannt, dass primäre Aminogruppen gegenüber Epoxidgruppen difunktionell sind und eine primäre Aminogruppe somit als zwei gegenüber Epoxidgruppen reaktive Gruppen zählt.

Die beiden Komponenten der Epoxidharz-Zusammensetzung werden jeweils in einem eigenen Gebinde gelagert. Weitere Bestandteile der Epoxidharz-Zusammensetzung können als Bestandteil der Harz- oder der Härter-Komponente vorhanden sein, wobei gegenüber Epoxidgruppen reaktive weitere Bestandteile bevorzugt ein Bestandteil der Härter-Komponente sind. Ein geeignetes Gebinde zum Lagern der Harz- oder der Härter-Komponente ist insbesondere ein Fass, ein Hobbock, ein Beutel, ein Eimer, eine Büchse, eine Kartusche oder eine Tube. Die Komponenten sind lagerfähig, das heisst, dass sie vor ihrer Anwendung während mehreren Monaten bis zu einem Jahr und länger aufbewahrt werden können, ohne dass sie sich in ihren jeweiligen Eigenschaften in einem für ihren Gebrauch relevanten Ausmass verändern. Zur Anwendung der Epoxidharz-Zusammensetzung werden die Harz- und die Härter-Komponente kurz vor oder während der Applikation miteinander vermischt. Das Mischungsverhältnis zwischen den beiden Komponenten wird bevorzugt so gewählt, dass die gegenüber Epoxidgruppen reaktiven Gruppen der Härter-Komponente in einem geeigneten Verhältnis zu den Epoxidgruppen der Harz-Komponente stehen, wie vorgängig beschrieben. In Gewichtsteilen liegt das Mischungsverhältnis zwischen der Harz-Komponente und der Härter-Komponente üblicherweise im Bereich von 1:10 bis 10:1.
Die Vermischung der beiden Komponenten erfolgt mittels eines geeigneten Verfahrens; sie kann kontinuierlich oder batchweise erfolgen. Falls das Vermischen vor der Applikation erfolgt, muss darauf geachtet werden, dass zwischen dem Vermischen der Komponenten und der Applikation nicht zu viel Zeit vergeht, da es dadurch zu Störungen, wie beispielsweise einem verlangsamten oder unvollständigen Aufbau der Haftung zum Substrat, kommen kann. Die Vermischung erfolgt insbesondere bei Umgebungstemperatur, welche typischerweise im Bereich von etwa 5 bis 50°C, bevorzugt bei etwa 10 bis 30°C, liegt.
Mit der Vermischung der beiden Komponenten beginnt die Aushärtung durch chemische Reaktion, wie vorgängig beschrieben. Die Aushärtung erfolgt insbesondere bei Umgebungstemperatur. Sie erstreckt sich typischerweise über einige Tage bis Wochen, bis sie unter den gegebenen Bedingungen weitgehend abgeschlossen ist. Die Dauer hängt unter anderem von der Temperatur, der Reaktivität der Bestandteile und deren Stöchiometrie sowie der Gegenwart von Beschleunigern ab.
Ein weiterer Gegenstand der Erfindung ist somit eine ausgehärtete Zusammensetzung erhalten aus der Aushärtung einer Epoxidharz-Zusammensetzung wie im vorliegenden Dokument beschrieben.

Die Applikation der Epoxidharz-Zusammensetzung erfolgt auf mindestens ein Substrat, wobei die Folgenden besonders geeignet sind:
- Glas, Glaskeramik, Beton, Mörtel, Backstein, Ziegel, Gips oder Natursteine wie Granit oder Marmor;
- Metalle oder Legierungen wie Aluminium, Eisen, Stahl oder Buntmetalle, oder oberflächenveredelte Metalle oder Legierungen wie verzinkte oder verchromte Metalle;
- Leder, Textilien, Papier, Holz, mit Harzen, beispielsweise Phenol-, Melamin-oder Epoxidharzen, gebundene Holzwerkstoffe, Harz-Textil-Verbundwerkstoffe oder weitere sogenannte Polymer-Composites;
- Kunststoffe, insbesondere Hart- oder Weich-PVC, ABS, Polycarbonat (PC), Polyamid (PA), Polyester, PMMA, Epoxidharze, PUR, POM, PO, PE, PP, EPM oder EPDM, wobei die Kunststoffe gegebenenfalls mittels Plasma, Corona oder Flammen oberflächenbehandelt sind;
- Faserverstärkte Kunststoffe, wie Kohlefaser-verstärkte Kunststoffe (CFK), Glasfaser-verstärkte Kunststoffe (GFK) oder Sheet Moulding Compounds (SMC);
- beschichtete Substrate, wie pulverbeschichtete Metalle oder Legierungen;
- Farben oder Lacke.

Die Substrate können bei Bedarf vor dem Applizieren der Epoxidharz-Zusammensetzung vorbehandelt werden. Derartige Vorbehandlungen umfassen insbesondere physikalische und/oder chemische Reinigungsverfahren, beispielsweise Schleifen, Sandstrahlen, Kugelstrahlen, Bürsten und/oder Abblasen, sowie weiterhin Behandeln mit Reinigern oder Lösemitteln oder das Aufbringen eines Haftvermittlers, einer Haftvermittlerlösung oder eines Primers.

Die beschriebene Epoxidharz-Zusammensetzung ist vorteilhaft verwendbar als Faserverbundmatrix für Faserverbundwerkstoffe (Composites) wie insbesondere CFK oder GFK, oder als Vergussmasse, Dichtstoff, Klebstoff, Belag, Beschichtung, Anstrich, Lack, Versiegelung, Grundierung oder Primer. Insbesondere verwendbar ist sie als Vergussmasse, beispielsweise als Elektrovergussmasse, oder als Klebstoff, insbesondere als Karrosserieklebstoff, Sandwichelementklebstoff, Halbschalenklebstoff für Rotorblätter von Windkraftanlagen, Brückenelementklebstoff oder Verankerungsklebstoff.
Insbesondere verwendbar ist sie weiterhin als Belag, Beschichtung, Anstrich, Lack, Versiegelung, Grundierung oder Primer für Bau- und Industrieanwendungen, insbesondere als Bodenbelag oder Bodenbeschichtung für Innenräume wie Büros, Industriehallen, Turnhallen oder Kühlräume, oder im Aussenbereich für Balkone, Terrassen, Parkdecks, Brücken oder Dächer, als Schutzbeschichtung für Beton, Zement, Metalle, Kunststoffe oder Holz, beispielsweise zur Oberflächenversiegelung von Holzkonstruktionen, Fahrzeugen, Ladeflächen, Tanks, Silos, Schächten, Rohrleitungen, Pipelines, Maschinen oder Stahlkonstruktionen, beispielsweise von Schiffen, Piers, Offshore-Plattformen, Schleusentoren, Wasserkraftwerken, Flussbauten, Schwimmbädern, Windkraftanlagen, Brücken, Kaminen, Kranen oder Spundwänden.
Insbesondere verwendbar ist sie weiterhin als Voranstrich, Haftanstrich, Korrosionsschutz-Primer oder zur Hydrophobierung von Oberflächen.
Auf die vollständig oder teilweise ausgehärtete Epoxidharz-Zusammensetzung kann insbesondere bei ihrer Verwendung als Beschichtung, Belag oder Anstrich eine weitere Beschichtung, ein weiterer Belag, oder ein weiterer Anstrich appliziert werden, wobei es sich bei dieser weiteren Schicht ebenfalls um eine Epoxidharz-Zusammensetzung handeln kann, aber auch um ein anderes Material, insbesondere um eine Polyurethan- oder Polyharnstoff-Beschichtung.

Besonders vorteilhaft wird die beschriebene Epoxidharz-Zusammensetzung als Beschichtung verwendet.
Ein weiterer Gegenstand der Erfindung ist dementsprechend eine Beschichtung, enthaltend eine Epoxidharz-Zusammensetzung wie vorgängig beschrieben.

Als Beschichtung werden dabei flächig aufgebrachte Beläge aller Art verstanden, insbesondere Anstriche, Lacke, Versiegelungen, Grundierungen oder Primer, wie vorgängig beschrieben, oder Bodenbeläge oder Schutzbeschichtungen, insbesondere auch solche für schweren Korrosionsschutz. Besonders vorteilhaft wird die beschriebene Epoxidharz-Zusammensetzung in emissionsarmen Beschichtungen mit Öko-Gütesiegeln, beispielsweise nach Emicode (EC1 Plus), AgBB, DIBt, Der Blaue Engel, AFSSET, RTS (M1) und US Green Building Council (LEED), verwendet.

Als Beschichtung wird die Epoxidharz-Zusammensetzung vorteilhaft in einem Verfahren zum Beschichten verwendet, wobei sie eine flüssige Konsistenz mit niedriger Viskosität und guten Verlaufseigenschaften aufweist und insbesondere als selbstverlaufende oder thixotropierte Beschichtung auf überwiegend ebene Flächen oder als Anstrich appliziert wird. Bevorzugt weist die Epoxidharz-Zusammensetzung bei dieser Applikation unmittelbar nach dem Vermischen der Harz- und der Härter-Komponente eine Viskosität, gemessen bei 20°C, im Bereich von 300 bis 4'000 mPa·s, bevorzugt im Bereich von 300 bis 2'000 mPa·s, besonders bevorzugt im Bereich von 300 bis 1'500 mPa·s, auf. Die vermischte Zusammensetzung wird innerhalb der Verarbeitungszeit flächig als dünner Film mit einer Schichtdicke von typischerweise etwa 50 µm bis etwa 5 mm auf ein Substrat appliziert, typischerweise bei Umgebungstemperatur. Die Applikation erfolgt insbesondere durch Aufgiessen auf das zu beschichtende Substrat und anschliessendem gleichmässigem Verteilen mit Hilfe beispielsweise eines Rakels oder einer Zahntraufel. Die Applikation kann auch mit einem Pinsel oder Roller oder als Spritzapplikation erfolgen, beispielsweise als Korrosionsschutzbeschichtung auf Stahl.
Bei der Aushärtung entstehen typischerweise weitgehend klare, glänzende und nichtklebrige Filme von hoher Härte, welche eine gute Haftung zu verschiedensten Substraten aufweisen.

Aus der Anwendung der Epoxidharz-Zusammensetzung entsteht ein Artikel umfassend die ausgehärtete Zusammensetzung aus der Aushärtung der beschriebenen Epoxidharz-Zusammensetzung. Die ausgehärtete Zusammensetzung liegt dabei insbesondere in Form einer Beschichtung vor.

Die beschriebene Epoxidharz-Zusammensetzung zeichnet sich durch vorteilhafte Eigenschaften aus. Sie ist niedrigviskos und geruchsarm und härtet auch bei feucht-kaltenBedingungen schnell und weitgehend ohne Blushing-Effekte aus, sogar mit geringen Anteilen oder ganz ohne die Verwendung von Verdünnern, und insbesondere auch ohne die Verwendung von leichtflüchtigen, geruchsintensiven Aminen. Bei der flächigen Verwendung als Beschichtung entstehen klare, nichtklebrige Filme von hoher Härte und hoher Oberflächenqualität, welche unter Lichteinfluss kaum vergilben. Mit der beschriebenen Epoxidharz-Zusammensetzung sind insbesondere emissionsarme Epoxidharz-Produkte zugänglich, welche die Bedingungen für viele Öko-Gütesiegel erfüllen und gleichzeitig hohen Ansprüchen bezüglich Arbeitssicherheit, Verarbeitungs-und Gebrauchseigenschaften genügen.

Ein weiterer Gegenstand der Erfindung ist die Verwendung eines Amins der Formel (I), wie vorgängig beschrieben, als Bestandteil eines Härters für Epoxidharze, wobei für den Fall, dass n für 0 steht, zusätzlich mindestens ein Amin **A,** wie vorgängig beschrieben, vorhanden ist.

Ein weiterer Gegenstand der Erfindung ist eine Methode zum Verdünnen eines Härters für Epoxidharze und/oder einer Epoxidharz-Zusammensetzung, indem ein Amin der Formel (I), wie vorgängig beschrieben, zugegeben wird.
Der Härter für Epoxidharze beziehungsweise die Epoxidharz-Zusammensetzung enthält dabei insbesondere ein mindestens drei Aminwasserstoffe aufweisendes Addukt aus mindestens einem Polyamin und mindestens einem Epoxid, wie vorgängig beschrieben.
Bevorzugt ist das Addukt entweder ein Addukt aus mindestens einem Polyamin und mindestens einem aromatischen Monoepoxid, welche im Molverhältnis von ungefähr 1/1 umgesetzt sind, oder ein Addukt aus mindestens einem Polyamin und mindestens einem aromatischen Diepoxid, welche im Molverhältnis von ungefähr 2/1 umgesetzt sind. Während der Umsetzung kann das Polyamin im Überschuss vorhanden gewesen und nach der Umsetzung mittels Destillation entfernt worden sein. Für ein solches Addukt ist das aromatische Monoepoxid bevorzugt ein Kresylglycidylether, insbesondere orto-Kresylglycidylether, und das Polyamin ist bevorzugt 1,2-Propylendiamin oder MPMD. Das aromatische Diepoxid ist insbesondere ein Bsiphenol-A- oder -F- oder -A/F-Diglycidylether oder ein Resorcinol-Diglycidylether, insbesondere ein kommerziel erhältliches Flüssigkharz, und das Polyamin ist bevorzugt 1,2-Ethylendiamin oder 1,2-Propylendiamin.
Nach dem Verdünnen weist der Härter insbesondere eine Viskosität, gemessen bei 20 °C, im Bereich von 100 bis 4'000 mPa·s, bevorzugt im Bereich von 100 bis 2'000 mPa·s, besonders bevorzugt im Bereich von 100 bis 1'500 mPa·s auf.
Nach dem Verdünnen weist die Epoxidharz-Zusammensetzung unmittelbar nach dem Vermischen mit dem Amin der Formel (I) insbesondere eine Viskosität, gemessen bei 20 °C, im Bereich von 300 bis 4'000 mPa·s, bevorzugt im Bereich von 300 bis 2'000 mPa·s, besonders bevorzugt im Bereich von 300 bis 1'500 mPa·s auf.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.
"AHEW" steht für das Aminwasserstoff-Equivalentgewicht.
"EEW" steht für das Epoxid-Equivalentgewicht.
Als "Normklima" wird eine Temperatur von 23±1 °C und eine relative Luftfeuchtigkeit von 50±5% bezeichnet. "NK" steht für "Normklima".

### Beschreibung der Messmethoden:

**Infrarotspektren** (FT-IR) wurden als unverdünnte Filme auf einem mit horizontaler ATR-Messeinheit mit ZnSe-Kristall ausgestatteten FT-IR Gerät 1600 von Perkin-Elmer gemessen; die Absorptionsbanden sind in Wellenzahlen (cm⁻¹) angegeben (Messfenster: 4000-650 cm⁻¹).
**¹H-NMR-Spektren** wurden auf einem Spektrometer des Typs Bruker Ascend 400 bei 400.14 MHz gemessen; die chemischen Verschiebungen δ sind angegeben in ppm relativ zu Tetramethylsilan (TMS). Echte und Pseudo-Kopplungsmuster wurden nicht unterschieden.
**Gaschromatogramme** (GC) wurden gemessen im Temperaturbereich von 60 bis 320 °C mit einer Aufheizrate von 15 °C/min und 10 min Verweilzeit bei 320 °C. Die Injektortemperatur betrug 250 °C. Es wurde eine Zebron ZB-5 Säule verwendet (L = 30 m, ID = 0.25 mm, dj = 0.5 µm) bei einem Gasfluss von 1.5 ml/Min. Die Detektion erfolgte mittels Flammenionisation (FID).
Die **Viskosität** von höherviskosen Proben (oberhalb von 150 mPa·s)wurde auf einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 s⁻¹) gemessen.
Die **Viskosität** von niedrigviskosen Proben (unterhalb von 150 mPa·s)wurde auf einem thermostatisierten Kegel-Platten Rheometer Anton Paar Physica MCR 300 (Kegeldurchmesser 25 mm, Kegelwinkel 2°, Kegelspitze-PlattenAbstand 0.05 mm, Scherrate 100 s⁻¹) gemessen.
Die **Aminzahl** wurde durch Titration bestimmt (mit 0.1N HClO₄ in Essigsäure gegen Kristallviolett).

### Verwendete Substanzen:

| | |
|---|---|
| Araldite^{®} GY 250: | Bisphenol-A-Diglycidylether, EEW ca. 187.5 g/Eq (von Huntsman) |
| Araldite^{®} DY-E: | Monoglycidylether von C₁₂- bis C₁₄-Alkoholen, EEW ca. 290 g/Eq (von Huntsman) |
| EP-Addukt 2: | Umsetzungsprodukt aus 1,5-Diamino-2-methylpentan und Araldite^{®} DY-K, wie nachfolgend beschrieben; AHEW ca. 106.5 g/Eq; Viskosität (20°C) 13'000 mPa·s |
| EP-Addukt 3: | Umsetzungsprodukt aus 1,2-Propylendiamin und Araldite^{®} DY-K, wie nachfolgend beschrieben; AHEW ca. 90.0 g/Eq; Viskosität (20°C) 23'000 mPa·s |
| Araldite^{®} DY-K: | Kresylglycidylether, EEW ca. 182 g/Eq (von Huntsman) |
| Gaskamine^{®} 240: | Styrolisiertes 1,3-Bis(aminomethyl)benzol; AHEW 103 g/Eq; Viskosität (20°C) 165 mPa·s (von Mitsubishi Gas Chemical) |
| Jeffamine^{®} D-230: | Polyoxypropylendiamin mit mittlerem Molekulargewicht ca. 240 g/mol, AHEW ca. 60 g/Eq (von Huntsman) |
| Ancamine^{®} K 54: | 2,4,6-Tris(dimethylaminomethyl)phenol (von Air Products) |

Das **EP-Addukt 2** wurde hergestellt, indem 4.65 kg 1,5-Diamino-2-methylpentan (Dytek^{®} A von Invista) unter Stickstoffatmosphäre vorgelegt, auf 70 °C aufgewärmt und dann unter gutem Rühren langsam mit 1.83 kg Araldite^{®} DY-K versetzt wurde, wobei die Temperatur der Reaktionsmischung 70 bis 80 °C betrug. Nach 1 Stunde bei 80°C wurde die Reaktionsmischung abgekühlt und die flüchtigen Bestandteile destillativ mittels Dünnschichtverdampfer (0.5-1 mbar, Manteltemperatur 160 °C) entfernt.

Das **EP-Addukt 3** wurde hergestellt, indem 4.15 kg 1,2-Propylendiamin unter Stickstoffatmosphäre vorgelegt, auf 70 °C aufgewärmt und dann unter gutem Rühren langsam mit 2.93 kg Araldite^{®} DY-K versetzt wurde, wobei die Temperatur der Reaktionsmischung 70 bis 80 °C betrug. Nach 1 Stunde bei 80°C wurde die Reaktionsmischung abgekühlt und die flüchtigen Bestandteile destillativ mittels Dünnschichtverdampfer (0.5-1 mbar, Manteltemperatur 115 °C) entfernt.

### Herstellung von Aminen:

### Amin 1: N-Benzyl-1,2-ethandiamin

In einem Rundkolben wurden 120.2 g (2 mol) 1,2-Ethylendiamin unter Stickstoffatmosphäre bei Raumtemperatur vorgelegt. Unter gutem Rühren wurde langsam eine Lösung aus 42.4 g (0.4 mol) Benzaldehyd in 800 ml Isopropanol dazugetropft und 2 Stunden nachgerührt. Die Reaktionsmischung wurde anschliessend bei einem Wasserstoff-Druck von 90 bar, einer Temperatur von 90 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65 °C eingeengt, wobei unreagiertes 1,2-Ethylendiamin und Isopropanol entfernt wurden. Die so erhaltene Reaktionsmischung war eine klare, leicht gelbliche Flüssigkeit mit einer Aminzahl von 668.4 mg KOH/g. 50 g dieser Reaktionsmischung wurden bei 80 °C unter Vakuum destilliert, wobei 31.3 g Destillat bei einer Dampftemperatur von 60 bis 65 °C und 0.06 bar aufgefangen wurden. Erhalten wurde eine farblose Flüssigkeit mit einer Viskosität von 8.3 mPa·s bei 20 °C, einer Aminzahl von 749.6 mg KOH/g und einer mittels GC bestimmten Reinheit von > 97 % (Retentionszeit 8.47 - 8.57 min), welche im Folgenden als Amin 1 eingesetzt wurde.
¹H-NMR (CDCl₃): 7.36-7.32 (m, 5 H, Ar-H), 3.79 (s, 2 H, Ar-CH₂), 2.80 (t, 2 H, C*H*₂NH₂), 2.68 (t, 2 H, NHC*H*₂CH₂), 1.28 (br s, 3 H NH und NH₂)
FT-IR: 3365, 3285, 3025, 2913, 2814, 1601, 1493, 1451, 1199, 1067, 1027, 801,731.

### Amin 2: N-(4-Methoxybenzyl)-1,2-ethandiamin

In einem Rundkolben wurden 120.2 g (2 mol) 1,2-Ethylendiamin unter Stickstoffatmosphäre bei Raumtemperatur vorgelegt. Unter gutem Rühren wurde langsam eine Lösung aus 54.4 g (0.4 mol) 4-Methoxybenzaldehyd (= Anisaldehyd) in 800 ml Isopropanol dazugetropft und 2 Stunden nachgerührt. Die Reaktionsmischung wurde anschliessend bei einem Wasserstoff-Druck von 90 bar, einer Temperatur von 85 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65 °C eingeengt, wobei unreagiertes 1,2-Ethylendiamin und Isopropanol entfernt wurden. Die so erhaltene Reaktionsmischung war eine klare, gelbliche Flüssigkeit.
62.7 g dieser Reaktionsmischung wurden bei 110 °C unter Vakuum destilliert, wobei 48.9 g Destillat bei einer Dampftemperatur von 90 bis 92 °C und 0.024 bar aufgefangen wurden. Erhalten wurde eine farblose Flüssigkeit mit einer Viskosität von 22 mPa·s bei 20 °C, einer Aminzahl von 615.1 mg KOH/g und einer mittels GC bestimmten Reinheit von > 97 % (Retentionszeit 10.69 min), welche im Folgenden als Amin **2** eingesetzt wurde.
¹H-NMR (CDCl₃): 7.22 (d, 2 H, Ar-H), 6.85 (d, 2 H, Ar-H), 3.78 (s, 3 H, OCH₃), 3.72 (d, 2 H, Ar-C*H*₂NH), 2.79 (t, 2 H, C*H*₂NH₂), 2.66 (t, 2 H, NHC*H*₂CH₂), 1.29 (br s, 3 H NH und NH₂).
FT-IR: 3285, 2931, 2832, 1610, 1584, 1509, 1461, 1441, 1299, 1248, 1173, 1106, 1031, 808.

### Amin 3: N-Benzyl-1,3-propandiamin (Vergleich)

In einem Rundkolben wurden 148.3 g (2 mol) 1,3-Propandiamin unter Stickstoffatmosphäre bei Raumtemperatur vorgelegt. Unter gutem Rühren wurde langsam eine Lösung aus 42.4 g (0.4 mol) Benzaldehyd in 800 ml Isopropanol dazugetropft und 2 Stunden nachgerührt. Die Reaktionsmischung wurde anschliessend bei einem Wasserstoff-Druck von 90 bar, einer Temperatur von 90 °C und einem Fluss von 5 ml/min auf einer kontinuierlich arbeitenden Hydrierapparatur mit Pd/C-Festbettkatalysator hydriert. Zur Reaktionskontrolle wurde mittels IR-Spektroskopie überprüft, ob die Iminbande bei ca. 1665 cm⁻¹ verschwunden war. Darauf wurde die hydrierte Lösung am Rotationsverdampfer bei 65 °C eingeengt, wobei unreagiertes 1,3-Propandiamin und Isopropanol entfernt wurden. Die so erhaltene Reaktionsmischung war eine klare, leicht gelbliche Flüssigkeit mit einer Aminzahl von 569 mg KOH/g. 50 g dieser Reaktionsmischung wurden bei 90 °C unter Vakuum destilliert, wobei 33.8 g Destillat bei einer Dampftemperatur von 68 bis 73 °C und 0.06 bar aufgefangen wurden. Erhalten wurde eine farblose Flüssigkeit mit einer Viskosität von 10.8 mPa·s bei 20 °C, einer Aminzahl von 682 mg KOH/g und einer mittels GC bestimmten Reinheit von > 97 % (Retentionszeit 9.39 - 9.46 min), welche im Folgenden als Amin **3** zu Vergleichszwecken eingesetzt wurde.

### Herstellung von Härtern und Epoxidharz-Zusammensetzungen

Für jedes Beispiel wurden die in den Tabellen 1 bis 2 angegebenen Inhaltsstoffe in den angegebenen Mengen (in Gewichtsteilen) der Härter-Komponente mittels eines Zentrifugalmischers (SpeedMixer™ DAC 150, FlackTek Inc.) vermischt und unter Ausschluss von Feuchtigkeit aufbewahrt.
Ebenso wurden die in den Tabellen 1 bis 2 angegebenen Inhaltsstoffe der Harz-Komponente verarbeitet und aufbewahrt.
Anschliessend wurden die beiden Komponenten jeder Zusammensetzung mittels des Zentrifugalmischers zu einer homogenen Flüssigkeit verarbeitet und diese unverzüglich folgendermassen geprüft:
10 Minuten nach dem Vermischen wurde die Viskosität bei 20°C bestimmt **("Viskosität (10')").**

Ein erster Film wurde in einer Schichtdicke von 500 µm auf eine Glasplatte aufgezogen und dieser Normklima gelagert bzw. ausgehärtet. An diesem Film wurde die **Königshärte** (Pendelhärte nach König, gemessen nach DIN EN ISO 1522) nach 1 Tag ("Königshärte (1d NK)"), nach 2 Tagen ("Königshärte (2d NK)"), nach 4 Tagen ("Königshärte (4d NK)"), nach 7 Tagen ("Königshärte (7d NK)") und nach 14 Tagen ("Königshärte (14d NK)") bestimmt. Nach 14 Tagen wurde der Aspekt des Films beurteilt (in der Tabelle mit "Aspekt (NK)" bezeichnet). Als "schön" wurde dabei ein Film bezeichnet, welcher klar war und eine glänzende und nichtklebrige Oberfläche ohne Struktur aufwies. Als "Struktur" wird dabei jegliche Art von Zeichnung oder Muster auf der Oberfläche bezeichnet.

Ein zweiter Film wurde in einer Schichtdicke von 500 µm auf eine Glasplatte aufgezogen und dieser unmittelbar nach dem Applizieren während 7 Tagen bei 8 °C und 80 % relativer Feuchtigkeit und anschliessend während 3 Wochen im NK gelagert, beziehungsweise ausgehärtet. 24 Stunden nach der Applikation wurde ein Flaschendeckel aus Polypropylen auf den Film aufgesetzt, unter welchem ein feuchtes Schwämmchen platziert war. Nach weiteren 24 Stunden wurde das Schwämmchen und der Deckel entfernt und an einer neuen Stelle des Films platziert, wo es nach 24 Stunden wieder entfernt und neu platziert wurde, insgesamt 4 mal. Anschliessend wurde der Aspekt dieses Films beurteilt (in den Tabellen mit "Aspekt (8°/80%)" bezeichnet), auf die gleiche Weise wie für den Aspekt (NK) beschrieben. Dabei wurde jeweils auch die Anzahl Markierungen angegeben, die im Film durch das feuchte Schwämmchen und/ oder den aufgesetzten Deckel sichtbar waren. An den so ausgehärteten Filmen wurde wiederum die Königshärte bestimmt, jeweils nach 7 Tagen bei 8 °C und 80 % relativer Feuchtigkeit ("Königsh. (7d 8°/80%)"), dann nach weiteren 2 Tagen im NK ("Königsh. (+2d NK)"), 7 Tagen im NK ("Königsh. (+7d NK)") und 14d im NK ("Königsh. (+14d NK)").
Als Mass für die Vergilbung wurde weiterhin die Farbveränderung nach Belastung in einem Bewitterungstester bestimmt. Dazu wurde ein weiterer Film in einer Schichtdicke von 500 µm auf eine Glasplatte aufgezogen und dieser im Normklima während 2 Wochen gelagert bzw. ausgehärtet und anschliessend in einem Bewitterungstester des Typs Q-Sun Xenon Xe-1 mit optischem Filter Q-SUN Daylight-Q und einer Xenon Lampe mit einer Lichtstärke von 0.51 W/m² bei 340 nm bei einer Temperatur von 65°C während 72 Stunden belastet **(Q-Sun (72h)).** Anschliessend wurde der Farbunterschied ΔE des so belasteten Films im Vergleich zum entsprechenden nicht belasteten Film mittels einem Colorimeter NH310 von Shenzen 3NH Technology Co. LTD, ausgerüstet mit Silicon Photoelectric Diode Detector, Light Source A, Color Space Measurement Interface CIE L*a*b*C*H*, bestimmt. ΔE-Werte von 0.5 bis 1.5 stehen dabei für einen geringfügigen Farbunterschied, 1.5 bis 3 für einen merklichen Farbunterschied, 3 bis 6 für einen deutlich sichtbaren Farbunterschied und mehr als 6 für einen grossen Farbunterschied.
Die Resultate sind in den Tabellen 1 bis 2 angegeben.
Bei den Epoxidharz-Zusammensetzungen **EZ-1** bis **EZ**-7 handelt es sich um erfindungsgemässe Beispiele. Bei den Epoxidharz-Zusammensetzungen ***Ref-1*** bis ***Ref***-***5*** handelt es sich um Vergleichsbeispiele.

**Tabelle 1: Zusammensetzung und Eigenschaften von EZ-1 bis EZ-4 und Ref-1 und Ref-2.**

| **Beispiel** | | | ***EZ-1*** | ***EZ-2*** | ***EZ-3*** | ***EZ-4*** | ***Ref-1*** | ***Ref-2*** |
|---|---|---|---|---|---|---|---|---|
| **Harz-Komponente:** | | | | | | | | |
| | Araldite^{®} GY-250 | | 167.2 | 167.2 | 167.2 | 167.2 | 167.2 | 167.2 |
| | Araldite^{®} DY-E | | 31.8 | 31.8 | 31.8 | 31.8 | 31.8 | 31.8 |
| **Härter-Komponente:** | | | | | | | | |
| | | Amin | **1** | **1** | **1** | **2** | **3** | |
| | | | 25.0 | 25.0 | 25.0 | 30.9 | 27.4 | - |
| | Gaskamine^{®} 240 | | - | - | - | - | - | 51.5 |
| | EP-Addukt-2 | | 53.3 | 53.3 | - | - | - | - |
| | EP-Addukt-3 | | - | - | 45.0 | 45.0 | 45.0 | 45.0 |
| | Jeffamine^{®} D-230 | | - | - | - | - | - | - |
| | Salicylsäure | | 1.3 | - | - | - | - | - |
| | Ancamine^{®} K 54 | | 1.3 | - | - | - | - | - |
| Viskosität (10') [Pa·s] | | | 1.53 | 1.39 | 1.33 | 2.16 | 1.39 | 1.66 |
| Königshärte [s] | | (1d NK) | 101 | 83 | 74 | 91 | 62 | 43 |
| | | (2d NK) | 144 | 125 | 134 | 140 | 109 | 87 |
| | | (4d NK) | 168 | 151 | 170 | 171 | 147 | 134 |
| | | (7d NK) | 177 | 170 | 188 | 188 | 171 | 148 |
| | | (14d NK) | 178 | 170 | 195 | 197 | 187 | 175 |
| Aspekt (NK) | | | schön | schön | schön | schön | schön | schön |
| Q-Sun (72h) ΔE | | | 10.3 | 4.3 | 2.6 | 3.8 | 3.7 | 5.0 |
| Königsh. [s] | | (7d 8°/80%) | 26 | 27 | 38 | 73 | 43 | 14 |
| | | (+2d NK) | 59 | 92 | 153 | 161 | 132 | 119 |
| | | (+7d NK) | 167 | 98 | 174 | 172 | 173 | 157 |
| | | (+14d NK) | 172 | 170 | 193 | 200 | 186 | 176 |
| Aspekt (8°/80%) | | | schön | schön | schön | schön | schön | schön |
| Anzahl Markierungen | | | 1 | 1 | 1 | 1 | 3 | 1 |

**Tabelle 2: Zusammensetzung und Eigenschaften von EZ-5 und EZ-7 und Ref-3 bis Ref-5.**

| **Beispiel** | | | ***EZ-5*** | ***EZ-6*** | ***Ref-3*** | ***Ref-4*** | ***EZ-7*** | ***Ref-5*** |
|---|---|---|---|---|---|---|---|---|
| **Harz-Komponente:** | | | | | | | | |
| | Araldite^{®} GY-250 | | 167.2 | 167.2 | 167.2 | 167.2 | 167.2 | 167.2 |
| | Araldite^{®} DY-E | | 31.8 | 31.8 | 31.8 | 31.8 | 31.8 | 31.8 |
| **Härter-Komponente:** | | | | | | | | |
| | | Amin | **1** | **1** | **3** | **1** | **2** | **3** |
| | | | 45.1 | 40.1 | 43.8 | 50.1 | 61.8 | 54.7 |
| | Jeffamine^{®} D-230 | | 6.0 | 12.0 | 12.0 | - | - | - |
| | Viskosität (10') [Pa·s] | | 0.29 | 0.30 | 0.30 | 0.33 | 0.45 | 0.36 |
| Königshärte [s] | | (1d NK) | 56 | 43 | 25 | 34 | 88 | 38 |
| | | (2d NK) | 71 | 109 | 52 | 79 | 123 | 53 |
| | | (4d NK) | 100 | 147 | 99 | 136 | 137 | 70 |
| | | (7d NK) | 136 | 154 | 99 | 163 | 145 | 85 |
| | | (14d NK) | 144 | 166 | 119 | 167 | 146 | 101 |
| Aspekt (NK) | | | schön | schön | sehr trüb | schön | schön | sehr trüb |
| Q-Sun (72h) ΔE | | | 3.2 | 3.0 | 12.6 | 3.4 | 9.3 | 13.2 |
| Königsh. [s] | | (7d 8°/80%) | 18 | 24 | 24 | 35 | 54 | 27 |
| | | (+2d NK) | 119 | 67 | 63 | 110 | 106 | 63 |
| | | (+7d NK) | 153 | 76 | 88 | 143 | 116 | 106 |
| | | (+14d NK) | 160 | 78 | 106 | 151 | 123 | 109 |
| Aspekt (8°/80%) | | | schön | schön | sehr trüb | leicht trüb | schön | sehr trüb |
| Anzahl Markierungen | | | 1 | 1 | 2 | 1 | keine | 2 |

## Patentansprüche

1. Epoxidharz-Zusammensetzung umfassend
- eine Harz-Komponente enthaltend mindestens ein Epoxidharz, und
- eine Härter-Komponente enthaltend mindestens ein Amin der Formel (I), 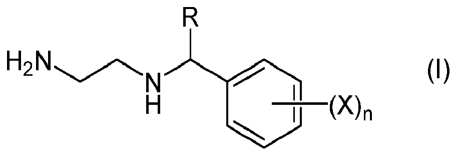 wobei
n für 0 oder 1 oder 2 oder 3 steht,
R für einen Wasserstoff-Rest oder für einen Kohlenwasserstoff-Rest mit 1 bis 6 C-Atomen steht, und
X für gleiche oder verschiedene Reste ausgewählt aus der Gruppe bestehend aus Alkyl, Alkoxy und Dialkylamino mit jeweils 1 bis 18 C-Atomen steht,
wobei die Härter-Komponente für den Fall, dass n für 0 steht, zusätzlich mindestens ein Amin **A** mit mindestens drei Aminwasserstoffen und einem Molekulargewicht von mindestens 200 g/mol, welches nicht der Formel (I) entspricht, enthält.

2. Epoxidharz-Zusammensetzung gemäss Anspruch 1, **dadurch gekennzeichnet, dass** R für einen Wasserstoff-Rest oder für Methyl steht.

3. Epoxidharz-Zusammensetzung gemäss einem der Ansprüche 1 oder 2, **dadurch gekennzeichnet, dass** R für einen Wasserstoff-Rest und n für 0 stehen.

4. Epoxidharz-Zusammensetzung gemäss einem der Ansprüche 1 oder 2, **dadurch gekennzeichnet, dass** R für einen Wasserstoff-Rest, n für 1 und X für Methoxy oder Dimethylamino in para-Stellung stehen.

5. Epoxidharz-Zusammensetzung gemäss einem der Ansprüche 1 bis 4, **dadurch gekennzeichnet, dass** das Amin der Formel (I) erhalten wird aus der reduktiven Alkylierung von 1,2-Ethylendiamin mit mindestens einem Aldehyd oder Keton der Formel (II) und Wasserstoff.

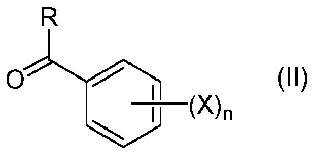

6. Epoxidharz-Zusammensetzung gemäss Anspruch 5, **dadurch gekennzeichnet, dass** 1,2-Ethylendiamin gegenüber den Carbonylgruppen des Aldehyds oder Ketons der Formel (II) im stöchiometrischen Überschuss eingesetzt und der Überschuss nach der Reduktion destillativ entfernt wird.

7. Epoxidharz-Zusammensetzung gemäss einem der Ansprüche 1 bis 6, **dadurch gekennzeichnet, dass** das Amin **A** ein Addukt aus mindestens einem Polyamin mit 2 bis 12 C-Atomen und mindestens einem Epoxid ist.

8. Epoxidharz-Zusammensetzung gemäss Anspruch 7, **dadurch gekennzeichnet, dass** das Epoxid ein aromatisches Monoepoxid ist, wobei das Polyamin und das aromatische Monoepoxid ungefähr im Molverhältnis 1/1 umgesetzt sind.

9. Epoxidharz-Zusammensetzung gemäss Anspruch 7, **dadurch gekennzeichnet, dass** das Epoxid ein aromatisches Diepoxid ist, wobei das Polyamin und das aromatische Diepoxid ungefähr im Molverhältnis 2/1 umgesetzt sind.

10. Epoxidharz-Zusammensetzung gemäss einem der Ansprüche 1 bis 9, **dadurch gekennzeichnet, dass** die Härter-Komponente 5 bis 65 Gewichts-% Amin der Formel (I) enthält.

11. Beschichtung enthaltend eine Epoxidharz-Zusammensetzung gemäss einem der Ansprüche 1 bis 10.

12. Ausgehärtete Zusammensetzung erhalten aus der Aushärtung einer Epoxidharz-Zusammensetzung gemäss einem der Ansprüche 1 bis 10 oder einer Beschichtung gemäss Anspruch 11.

13. Härter-Komponente enthaltend
- mindestens ein Amin der Formel (I), 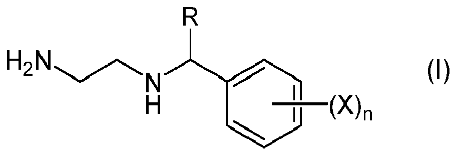 wobei
n für 0 oder 1 oder 2 oder 3 steht,
R für einen Wasserstoff-Rest oder für einen Kohlenwasserstoff-Rest mit 1 bis 6 C-Atomen steht, und
X für gleiche oder verschiedene Reste ausgewählt aus der Gruppe bestehend aus Alkyl, Alkoxy und Dialkylamino mit jeweils 1 bis 18 C-Atomen steht,
- und mindestens ein Amin **A** mit mindestens drei Aminwasserstoffen und einem Molekulargewicht von mindestens 200 g/mol, welches nicht der Formel (I) entspricht.

14. Verwendung eines Amins der Formel (I) wie in einem der Ansprüche 1 bis 6 beschrieben als Bestandteil eines Härters für Epoxidharze, wobei für den Fall, dass n für 0 steht, zusätzlich mindestens ein Amin **A** wie in einem der Ansprüche 7 bis 9 beschrieben vorhanden ist.

15. Methode zum Verdünnen eines Härters für Epoxidharze und/oder einer Epoxidharz-Zusammensetzung, **dadurch gekennzeichnet, dass** ein Amin der Formel (I) wie in einem der Ansprüche 1 bis 6 beschrieben zugegeben wird.
